# EUROPEAN PATENT APPLICATION

(11) **EP 2 932 971 A1**
(43) Date of publication of application: **21.10.2015**
(21) Application number: 15000954.6
(22) Date of filing: 06.03.2006
(51) Int. Cl.: A61K 31/54, A61K 31/445, A61K 9/08, A61K 9/51, A61L 31/00

(54) **KETAMINE FORMULATIONS**

(30) Priority: 04.03.2005 US 658207 P
(62) Divisional of application: 06736872.0
(71) Applicant: Otonomy, Inc., San Diego, CA 92121 (US)
(72) Inventor: LOBL, Thomas, Jay, Valencia, CA 91355-1995 (US); MCCORMACK, Stephen, Joseph, Claremont, CA 91711 (US); SCHLOSS, John, Vinton, Valencia, CA 91350 (US); NAGY, Anna Imola, Saugus, CA 91350 (US); PANANEN, Jacob, E., 306 Los Angeles, CA 90042 (US)
(74) Representative: Ali, Suleman

(57) **Abstract**

Formulations of ketamine for administration to the inner or middle ear.

## Description

This application claims the benefit of Serial No. 60/658,207 filed March 4, 2005. Serial No. 60/658,207 is incorporated herein by reference in its entirety.

### FIELD OF THE INVENTION

The invention relates to improved formulations of gacyclidine and other therapeutic agents useful for treating disorders of the middle and inner ear.

### BACKGROUND OF THE INVENTION

Many therapeutics when given systemically (*i.e.,* per-orally, intravenously, or intramuscularly) have side effects which prevent an optimal dose from being delivered. This is due to other body and nervous system cells being exposed to a full dose of the therapeutic obtained from the circulation while the targeted cells do not receive an optimal dose because of the systemic side effects elsewhere. When formulated correctly for tissue-specific drug delivery, a potent therapeutic, so delivered, will have minimal side effects and still be effective on the target nerve tissue.

As currently understood, tinnitus is frequently a symptom of a variety of neurological disorders, including damage to dental or facial nerves, temporomandibular joint (TMJ) disease, hypersensitivity to smell, taste and touch and damage to the auditory cortex or inferior colliculus. Tinnitus is also frequently comorbid with other manifestations of these underlying neurological disorders, such as Meniere's Disease, pain, anxiety, depression, and migraine headaches. As such, it is desirable to treat the precise location where the underlying neurological disorder occurs, which is not restricted to, but can include, the cochlea, auditory nerve, dental or facial nerves, and auditory cortex or inferior colliculus of the central nervous system. A patient recognizes tinnitus as an internal sound when there is no external sound. Clinically, the desirable treatment is to suppress the perception of inappropriate sound without side effects that prevent normal functioning at work and daily life.

Accordingly, the appropriate treatment necessarily will require the administration of a potent CNS active drug to quiet the inappropriate firing of the associated nerves that report the sound percept into the auditory cortex. Potent CNS active drugs that act to quiet the spontaneous and perhaps the tonic firing of specific nervous tissue without turning off other essential functions such as hearing or balance may be given for treatment of middle and inner ear disorders such as tinnitus. These drugs have specific limitations when given systemically because of their general activity on the entire nervous system. To overcome these systemic side effects and to get an improved therapeutic outcome while minimizing side effects, it is desirable to administer the drug into the site of tinnitus origin such as the middle or inner ear. Direct administration to the middle or inner ear allows the healthcare provider to optimize the therapeutic program to avoid or minimize tissue damage and side effects. Consequences of tissue damage in this organ resulting from an inappropriate dosing regimen are serious to the senses of hearing and balance and could include irreversible damage to the hearing related hair cells or the nerves, spiral ganglion, or vestibular system. In some cases, compounds administered into the inner ear or cochlea can drain into the cerebral spinal fluid (CSF). If a potent CNS active agent reaches the CSF it may have undesirable side effects because it could act on nerve cells which are not its target. Thus, there may be unintended effects on the brain and spinal cord. Damage to any of these cells and tissues would cause severe consequences to important senses such as hearing. In addition, compounds delivered to the middle ear in addition to the desired delivery of drug through the round window may also be absorbed partially into the exposed vascular system and get out into the general circulation or travel down the Eustachian tube to be absorbed in the mouth, throat, stomach or GI tract.

Accordingly, the administration method, location, device and formulation are all important for achieving a desirable outcome without undesirable side effects. Thus, there is a continuing need in the art for improved formulations of therapeutic agents that are specifically designed for delivery to the middle and inner ear or to other specific sites in need of therapy. This invention addresses this need.

### BRIEF DESCRIPTION OF THE DRAWINGS

FIG. 1. Graph showing a decrease in solution concentration of gacyclidine as a function of pH and the increase in solution concentration afforded by selected excipients at room temperature in glass vials.
FIG. 2. Graph showing adsorption of gacyclidine to polypropylene vials as a function of pH at 37°C and the moderating effects of selected excipients (1 mM total gacyclidine present; excess).
FIG. 3. Graph showing loss of 25 µM gacyclidine in a polypropylene vial as a function of pH with and without SOLUTOL^{®} (no excess gacyclidine).
FIG. 4. Graph showing chemical stability of gacyclidine base form and the chemical instability of the acid form at 54°C or 56°C.
FIG. 5. Graph showing increased chemical stability of gacyclidine acid form (pH 2) at lower temperatures. Note the surprisingly large temperature dependence of the decomposition rate.
FIG. 6. Graph showing that decomposition of gacyclidine is slower at pH 7.4 than at pH 5.5 or 6.0 in Ringer's Lactate at 37°C.
FIG. 7. Graph showing declining solution concentration of gacyclidine as the pH increases between pH 7.3 and 8.1 in Ringer's solution at 55°C and decreased production of piperidine at higher pH.
FIG. 8. Graph showing enhanced solution concentration of gacyclidine and chemical stability of gacyclidine by 0.3 % polysorbate 80 in Ringer's solution at 55°C between pH 6.7 and 7.2.
FIG. 9. Graph showing decreasing decomposition rate of gacyclidine at 55°C in Ringer's solution with 0.3 % polysorbate 80 added as pH increases between 6.7 and 7.2.

### DETAILED DESCRIPTION OF THE INVENTION

The invention provides formulations of gacyclidine and other drugs which are suitable for administration to the middle and inner ear. Such formulations can be used to treat disorders such as tinnitus, Meniere's disease, vertigo, middle and inner ear inflammation and infection, hearing loss induced by noise or certain kinds of trauma, and neurological damage resulting from physical (e.g., surgery) or chemical trauma.

### Definitions:

(a) A "carrier molecule" as used herein is a molecule which helps to retain an active agent in solution or suspension. Surfactants (amphipathic molecules which form a complex with water-insoluble or poorly soluble molecules and render the complex water soluble) are one category of carrier molecule.
(b) A "solubility enhancing reagent" increases the solubility of gacyclidine in a liquid formulation. Solubility enhancing reagents include carrier molecules, particularly surfactants, and organic solvents.
(c) "Solubility" as used herein is the property of a compound to dissolve into solution or be maintained in solution by a solubility enhancing reagent.
(d) "Solution concentration" as used herein is the concentration of gacyclidine per unit volume which is present in a randomly selected aliquot of a liquid formulation.
(e) "Chemical stability" as used herein means the substantial absence of gacyclidine decomposition as measured by piperidine formation. A gacyclidine formulation has increased chemical stability if it loses 10% or less of its initial gacyclidine solution concentration by decomposition to piperidine at 37°C within 4 days.
(f) An "excipient" as used herein is an additive to a formulation which helps to make the formulation pharmaceutically acceptable. Excipients include co-solvents (such as alcohols, glycols, DMSO, dimethylacetamide) to provide appropriate viscosity, minerals, antibacterial agents, and the like. Carrier molecules, including surfactants, can also be used as excipients.

Gacyclidine (1-(2-methyl-1-thiophen-2-yl-cyctohelxyl)-piperidine hydrochloride; GK11) is a well known compound. The chemical structure of the hydrochloride salt of gacyclidine is shown below: 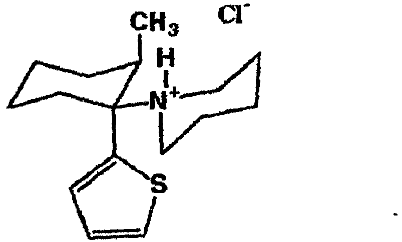 "Gacyclidine," as used herein, includes acid salt and basic forms and optical and geometric isomers of gacyclidine. "Gacyclidine derivatives" have minor modifications without changes to the core structural scaffold, such as an acetate derivative or addition of a methyl group to the nitrogen. "Gacyclidine analogs" have changes to the core scaffold, such as substitution of a carbon for a ring sulfur or nitrogen. See Geneste et al., Eur. J. Med. 14, 301-08, 1979). Gacyclidine derivatives and analogs containing a thiophen-2-yl ring would be enhanced by the formulation invention described herein.

Acid and base forms of gacyclidine can be prepared according to U.S. Patent 6,107,495. The chloride salt of the acid form of gacyclidine is very soluble in water or unbuffered physiologically acceptable solutions such as Ringer's solution and will form solutions containing up to 150 mg/ml of the chloride salt with a pH of about 4.4. The free base form of gacyclidine is poorly soluble in water and adheres to plastic surfaces. Addition of excess gacyclidine free base to a solution at physiological pH results in a final concentration of dissolved gacyclidine ≤ 0.02 mg/ml and a pH ≤ 7.7 the free base form of gacyclidine in Ringer's solution will react with dissolved carbon dioxide, to produce dissolved acid form of gacyclidine and a resultant concentration of approximately 0.02 mg/ml gacyclidine at pH 7.6. It is estimated by HPLC that 0.0006 mg/ml gacyclidine remained in solution at pH 9.5 (0.05 M CAPSO buffer; 0.1 M NaCl; 0.3 mg/mL gacyclidine added.) after sitting at room temperature for 2 days.

Drugs for injection into the inner or middle ear typically are formulated in a physiologically compatible solution and pH. However, at physiological pH (*e*.*g*., 6.8-7.4) and in various buffers at physiological pH gacyclidine is partially in the soluble salt form and partially in the uncharged free base form, which is poorly soluble in aqueous solvents. Thus, when formulated in aqueous solutions at physiological pH, gacyclidine forms soluble micro-aggregates which eventually adsorb to the container and/or coalesces into macro-aggregates and precipitates out of solution. Micro-aggregates can be filtered, centrifuged out of the solution during preparation of the formulation, or can adhere to the walls of the container or injection device containing the formulation. Under these conditions, the amount of drug delivered would be neither the desired amount nor the amount formulated for delivery. The manufacture of such formulations would be unreliable, producing different concentrations of drug in the formulation with each manufacturing run and would necessarily result in lowered efficacy. In addition, the drug becomes less soluble as the temperature increases. The pKa changes causing the acid-base equilibrium to shift to the basic form as the temperature increases.

Embodiments of this invention provide several solutions to this problem. In some embodiments, gacyclidine is formulated with selected solubility enhancing reagents (e.g., carrier molecules (including surfactants) and certain organic solvents) to provide both increased chemical stability and apparent solubility by holding the free base form in solution/clear suspension. In other embodiments gacyclidine is formulated as a lyophilized powder that is reconstituted with the proper vehicle to ensure rapid dissolution and the correct solution concentration, filterability and chemical stability. In other embodiments gacyclidine is formulated as the free base solid (with a specific shape for implantation such as a brick, sphere or pill shaped particle) which is dissolved slowly into physiological fluids or buffers to provide a therapeutic dosage. Gacyclidine formulated as described herein can be administered alone or in combination with one or more additional therapeutic agents as described below. Using formulations of the invention, a desired dose of gacyclidine can be delivered directly to the target tissue (for example, the cochlea) at a physiologically acceptable pH.

### Gacyclidine acidic and basic formulations comprising a solubility enhancing reagent

Gacyclidine in its acid or base form (or a mixture of the two) can be formulated in a vehicle with an acid (e.g. hydrochloric acid) or base (e.g. sodium bicarbonate or sodium hydroxide) added to bring the pH to the desired range. A significant and unexpected problem with acidic gacyclidine aqueous formulations, however, is the thermal chemical instability of the compound. In solution, gacyclidine decomposes to a variety of products including piperidine in a one to one ratio. Inclusion of a carrier molecule in an acidic gacyclidine formulation (i.e., pH < 7.0), typically at a concentration of between 0.1% to 10%, unexpectedly results in both increased solubility (which increases solution concentration) and increased chemical stability (see Example 6).

A significant problem with basic formulations is the insolubility of the basic form of gacyclidine in a variety of physiologically acceptable vehicles intended for solution delivery. Here, too, inclusion of a carrier molecule in a basic gacyclidine formulation (pH > 7), typically at a concentration of between 0.1 % to 10%, results in greater solubility of gacyclidine, prevents precipitation of gacyclidine from solution, reduces adsorption of gacyclidine onto container walls, and increases chemical stability of gacyclidine.

Optimal formulations comprise one or more solubility enhancing reagents. The amount of any particular solubility enhancing reagent to include in a gacyclidine formulation can be determined using routine methods, as described in Example 3.

It should be noted that equilibration of solution pH with ambient carbon dioxide in the air is a slow process that can take several hours. This is particularly noticeable when sodium bicarbonate, sodium carbonate or sodium hydroxide is used to adjust solution pH. The solubilizing enhancing reagent modifies solubility of gacyclidine in the vehicle. In some embodiments the solubility enhancing reagent is believed to reduce the polarity of the formulation mixture to foster drug interaction with the solubility enhancing reagent and to help keep the drug in solution. Examples of such solubility enhancing reagents include, but are not limited to, dimethyl acetamide, physiologically acceptable polyols (e.g., propylene glycol, glycerin), polyethylene glycol (PEG), and alcohols (e*.*g., ethyl alcohol).

In other embodiments the solubility enhancing reagent is a vehicle-soluble carrier molecule to which the drug will bind. The carrier molecule holds the bound drug in a homogeneous solution. The carrier molecule is then metabolized or excreted from the patient, and the drug exerts its biological effects. Carrier molecules useful in the invention include, but are not limited to, proteins which bind hydrophobic molecules (*e*.*g*., human or bovine serum albumin, fetuin, or any water-soluble protein which binds hydrophobic substances or which prevents nucleation and precipitation of water-insoluble substances); derivatized porphyrins or corins; negatively charged cyclic organic molecules with binding cavities ("crown compounds"); pharmaceutically acceptable phase transfer agents; ion exchange resins; diketopiperazines (DKP), such as TECHNOSPHERES^{®} (a succinyl group mono-derivatized on each lysine side chain of bis-lysine diketopiperazine), bis-glutamic acid DKP, bis-aspartic acid DKP and other DKPs which are derivatized to have a net negative charge(s) in the molecule; aqueous soluble lipids; micelles; liposomes (unilamellar and multilamellar vesicles); fatty acids and aqueous soluble lipids (e.g., cholic acid, chenic acid, deoxycholic acid, cardiolipin, cholylglycine, chenylglycine, deoxycholylglycine, cholyltaurine, chenyltaurine, deoxycholyltaurine); sulfatides (galactocerebrosides with a sulfate ester on the 3' position of the sugar); gangliosides; N-acetyl-D-neuraminic acid; phosphatidylinositol; soluble carbohydrates with net negative charges in the molecule (*e*.*g*., carboxymethyl cellulose); derivatized carbohydrates, and derivatives and mixtures of these carriers.

Other useful carrier molecules include, but are not limited to, a beta-cyclodextrin *(e.g.,* a sulfobutylether cyclodextrin, such as CAPTISOL^{®}) and surfactants such as polyoxyethylene esters of 12-hydroxystearic acid (e.g, SOLUTOL^{®} HS 15) and polysorbates, such as polysorbate 20, polysorbate 60, and polysorbate 80 *(e.g.,* TWEEN^{®}). Surfactants are particularly useful as carrier molecules for both acid and basic gacyclidine formulations. Addition of a surfactant is believed to adsorb or partially sequester the gacyclidine from the solvent (for example in micelles, liposomes and soluble coated aggregates) and thereby reduce its availability to bind to the surface of its storage container as a thin film, adhere to injection device components (syringe, needle, catheter, antibacterial filter, delivery pump reservoirs, pumping mechanisms and the like), or form visible or invisible micro-aggregates some of which, depending on size, can settle out of solution, be spun out of solution, or be trapped in filtration devices (such as antibacterial filters).

For example, addition of 2-20% (w/w) CAPTISOL^{®} (CyDex, Lenexa, KS), 0.1-20% TWEEN^{®} 80 (polysorbate 80, ICI Americas), or 0.1-20% SOLUTOL^{®} HS 15 (BASF) to solutions containing gacyclidine improves solubility. CAPTISOL^{®}, TWEEN^{®} 80, and SOLUTOL^{®} HS 15 can maintain 0.004, 0.01, and 0.02 grams of gacyclidine base in solution at room temperature per gram of carrier molecule respectively (see Example 3).

In one embodiment gacyclidine is present at a concentration from 1 nM to 5 mM and polysorbate 80 (*e*.*g*., TWEEN^{®} 80) is present at a concentration from 0.001% to 30% (weight/weight [w/w]). A preferred embodiment comprises 50 µM to 5 mM of gacyclidine and 0.1% to 20% (w/w) polysorbate 80 (*e*.*g*., TWEEN^{®} 80). Typically, the molar concentration of the carrier molecule is equal to or higher than the concentration of drug. Both gacyclidine and the carrier molecule can be formulated in a suitable vehicle, such as Ringer's solution at 290-300 mOsm.

In some embodiments one or more solubility enhancing reagents is combined with a vehicle and chemicals which will form a dimensionally rigid material that can be applied either as a finished aqueous gel or a liquid that gels (solidifies) following placement at the tissue site. Gels can be prepared from the vehicle-containing drug and solubility enhancing reagent mixed with a gel matrix, such as but not limited to hyaluronic acid, carboxymethyl cellulose or pleuronic acid. A gel so prepared will elute the drug slowly into the physiological fluids surrounding the delivery site.

### Excipients

Excipients can be included in formulations of the invention. These include, but are not limited to, d-alpha tocopheryl polyethylene glycol 1000 succinate (TPGS), cyclodextrins, ethylene glycol monostearate, glycerol stearate, glycerol mono- / di-caprylate / caprate, glyceryl behenate, glyceryl monooleate, glyceryl monostearate, glyceryl palmitostearate, lecithin, poloxamer 188, polyethylene glycols, polyglyceryl oleate, polyoxyl 40 stearate, polysorbate 20, polyoxyethylene sorbitan fatty acid esters, polyoxyethylene stearates, propylene glycol laurate, sodium lauryl sulfate, sodium stearyl fumarate, sorbitan esters (sorbitan fatty acid esters), sucrose octaacetate, stearic acid, and other pharmaceutically acceptable excipients useful for solubilizing or emulsifying water-insoluble drugs. The above excipient examples include both carrier molecules and surfactants. Other acceptable excipients not listed above include alcohols, glycols, glycerin, minerals, proteins and additives which help ensure the pharmaceutically acceptable nature of the formulation. See Pharmaceutical Excipients (Drugs and the Pharmaceutical Sciences, v. 94), David E. Bugay, Marcel Dekker AG, Basel, 1999; Handbook of Pharmaceutical Excipients, Raymond Rowe, Paul Sheskey, and Sian Owen, Eds., APhA Publications Fifth Edition, Washington, DC, 2006. One skilled in the art can choose from among these excipients to make formulations suitable for use in an animal or human. Such formulations would include, but not be limited to, soluble formulations suitable for parenteral administration. See Mark Gibson, in Pharmaceutical Preformulation and Formulation: A Practical Guide from Candidate Drug Selection to Commercial Dosage Form, Interpharm/CRC, Boca Raton, 2004.

For therapeutic use, the vehicle can be a pharmaceutically acceptable vehicle, such as Ringer's solution, Ringer's Lactate, artificial perilymph (see Konishi *et al.,* 1973), isotonic saline, and the like. Preferably the formulation has a pH between 5 and 10, with a pH between 6.5 and 8.5 being preferred; a physiologically acceptable osmolality between 280 and 310 mOsm with an osmolality of 290-300 being preferred; and preferably an ionic composition similar to that of perilymph when the formulation is prepared for middle and inner ear administration. The formulation delivered to the patient is preferably within the physiologically acceptable range of pH 6.8-7.4±0.5.

### Aqueous Vehicles

Formulations for internal administration have similar compositions to liquid parenteral formulations and are well known to those skilled in the art. The composition of inner ear fluid (perilymph) is unique and, because of the dynamics of sound transmission through this fluid, it is important that the drug supporting vehicle is compatible with it. The following are examples of pharmaceutically acceptable aqueous vehicles and their composition.

**Table 1**

| Solution Name | Concentration mMoL | | | | | pH | |
|---|---|---|---|---|---|---|---|
| | Na⁺ | K⁺ | Ca⁺⁺ | Cl⁻ | Lactate | | |
| Lactated Ringer's Injection USP (Ringer's Lactate) NDC 0074-7953-09 | 130 | 4 | 1.36 | 109 | 28 | 6.0 - 7.5 usual pH = 6.6 | |
| | | | | | | | |

| Solution Name | Concentration mMoL | | | | | pH | |
|---|---|---|---|---|---|---|---|
| | Na⁺ | K⁺ | Ca⁺⁺ | Cl⁻ | Lactate | | |
| Ringer's Injection USP (Ringer's solution) NDC 0338-0105-04 | 147 | 4 | 2.24 | 156 | 0 | 5.0 - 7.5 usual pH = 5.5 | |
| | | | | | | | |
| Human Perilymph [Data Summary from Wangemann&Schacht, 1996] | Concentration mMoL | | | | | Protein mg/dl | pH |
| | Na⁺ | K⁺ | Ca⁺⁺ | cr | Bicarbonate | | |
| | 148 | 4.2 | 1.3 | 119 | 21 | 178 | 7.3 |

One example of the composition of artificial perilymph from the literature is (modified from Konishi et al., Acta Otolaryng 76: 410-418):

| | | |
|---|---|---|
| 145mM | NaCl | 8.4738 g/L |
| 2.7mM | KCl | 0.2013 g/L |
| 2.0mM | MgSO₄ | 0.2408 g/L |
| 1.2mM | CaCl₂ | 0.1764 g/L |
| 5.0mM | HEPES | 1.1915 g/L |
| pH 7.3-7.4 | | |

### Osmolality

For inner ear administration the osmolality of the drug formulation is critical as the hair cells and associated supporting cells are very sensitive to the ionic strength of the formulation. The osmolality of commercially available Ringer's solution (Baxter Healthcare) and Ringer's Lactate (Abbott Laboratories) was determined by use of a freezing-point osmometer (Advanced Instruments Model 3MO Plus). The measured osmolality of Ringer's solution and Ringer's Lactate were 288 ± 2 and 255 ± 2 mOsm, respectively. The measured osmolality of human perilymph is 300 mOsm see Morris et al., Am J Otol 10, 148-9, 1989. The final osmolality of formulations for use in the middle or inner ear typically is adjusted to between 280 to 310 mOsm (preferably 290-300) by addition of sodium chloride, prior to use. The safety of this osmolality range has been confirmed by direct injection of appropriately formulated test solutions into the cochlea of guinea pigs.

### Storage of liquid formulations

### 1. Lyophilized formulations of acidic or free base forms of gacyclidine

Lyophilization of gacyclidine or a combination formulation provides a convenient way to store the formulated drug that minimizes chemical decomposition during long term storage. The acidic form of gacyclidine may be lyophilized from a frozen aqueous vehicle containing a bulking agent to aid reconstitution. Examples of bulking agents include but are not limited to lactose, mannitol, trehalose or glucose. A preferred vehicle for lyophilization of gacyclidine when it is not in combination with another therapeutic is water. When in combination with another therapeutic which is not sufficiently soluble to form a homogenous solution at the concentration needed for lyophilization in water, another suitable vehicle can be selected or excipients added to enhance the solubility of the mixture. Such vehicles would be known to someone skilled in the art. The reconstitution vehicle for such lyophilized formulations will contain the solubility enhancing reagent when the lyophilized gacyclidine formulation does not. Then when needed, the physician reconstitutes the lyophilized form of the drug in reconstitution vehicle that has the excipients and appropriate carrier molecules or surfactants required to help rapidly redissolve the solid, provide chemical stability and maintain the therapeutic in solution at the proper concentration either as a true solution of the gacyclidine acidic form or as a supported suspension of the acidic/basic form mixture or fully free base form. The pH of the reconstitution vehicle is adjusted appropriately to give the final desired pH needed for the application.

In other embodiments, the basic form of gacyclidine can be employed to improve the chemical stability in a lyophilized formulation. Such a formulation can easily be prepared by adding at least one equivalent of base to an aqueous solution of gacyclidine hydrochloride salt prior to lyophilization to convert it to the basic form (*e*.*g*., suitable neutralizing bases include but are not limited to sodium hydroxide or sodium carbonate). This drug-containing solution can also contain caking agents commonly used in lyophilized formulations, such as lactose, mannitol, trehalose or glucose. Solid excipients, such as cyclodextrins, can be added prior to lyophilization to chemically stabilize and aid the resolubilization (reconstitution) of the lyophilized gacyclidine base. Liquid additives, such as polysorbate 80, SOLUTOL^{®} HS 15 or cationic lipids, can be added as part of the reconstitution vehicle to the lyophilized formulation immediately prior to use. Sufficient acid, *e.g.,* hydrochloric acid, can be used in the solution, *e.g.,* Ringer's solution, used to reconstitute the dry powder, such that the desired pH is obtained upon dissolution of the dry formulation. The reconstitution pH will depend, in part, on the required solution stability of the gacyclidine formulation. As described in Example 6, the rate of gacyclidine decomposition will be slower at higher pH values. For longer storage of the reconstituted solution a higher pH would be desirable. If necessary, the pH of the formulation can be lowered by addition of acid, *e.g.,* hydrochloric acid, to physiologic pH, *e.g.,* pH 6.8 to 7.4, just prior to delivery to the intended site. The reconstituted, lyophilized product or solution formulation can be adjusted *in situ* by mixing a co-solvent stream inside a dual lumen device or immediately before loading the delivery device giving the final desired pH and formulation.

### 2. Storage containers

Polymeric surfaces, such as polypropylene, polyurethane, polyimide and polyvinyl chloride, have significant affinity for gacyclidine and can compete with formulation excipients and carrier molecules used to maintain gacyclidine solution concentration. Gacyclidine solutions formulated according to the invention preferably are maintained in acid-washed glass containers with an inert polymeric liner or cap e.g. polytetrafluoroethylene (PTFE). For similar reasons to the above, it is desirable to avoid contact of the formulation with containers, catheters, filters or other devices fabricated from selected polymeric materials, *e.g.*, polypropylene, polyurethane, polyvinyl chloride or silicone rubber. Where this is not possible, use of fluoropolymers, such as PTFE, as liners for the formulation contacting surfaces can help to minimize losses of gacyclidine, as can use of formulations, such as nanoparticle formulations, which can have higher affinity for gacyclidine than other polymers or surfactants used in delivering the drug.

### Formulations comprising solid gacyclidine free base

Other formulations of the invention include solid gacyclidine free base, which is a thermally more chemically stable form of gacyclidine especially at body temperature, *e.g.,* 37°C. The solid gacyclidine can be administered as the solid free base, for example, as a suspension in an aqueous vehicle or gel formulation (e.g. derived from but not limited to carboxymethyl cellulose, hayaluronic acid and pluronic acid and prepared as explained above except the drug formulation is pH adjusted to contain drug in the free base form rather than the soluble acidic form or a mixture of free base and acidic forms).

In other embodiments solid gacyclidine free base is adsorbed to a solid support, such as a collagen sponge or adsorbed or encapsulated in a particulate formulation, such as a nanoparticle formulation (e.g. erodible polylactate/polyglycolate polymer) or co-formulated with a polymeric material designed for slow release of the drug, such as a thin film non-erodible polymer matrix (e.g. silastic), through which the drug migrates to the surface and then is released to the target tissue (see below). Use of a solid support such as a thin film or coating results in slow release of gacyclidine as it is converted to the soluble acid salt form after it migrates to the polymeric surface or comes into contact with physiological fluids. Such slow release polymeric solid supports can be applied as thin films onto devices such as coated implanted electrodes, coated particles or materials where there is a drug core with a permeable thin film coating to modulate the rate of drug release. Examples of such coatings would include but not be limited to silastic or silicone rubber, polyurethane and polyvinyl chloride.

Solid supports such as nanoparticles comprising the solid gacyclidine free base and an erodible polymer carrier can be suspended in a pharmaceutically acceptable vehicle for therapeutic administration. Nanoparticles have the particular advantages of passing through antibacterial filters and ease of uptake into target cells.

In some embodiments, the solid free base form of gacyclidine is formulated as a suspension in an aqueous vehicle, adsorbed to a collagen sponge, or suspended in an aqueous gel formulation. An aqueous solution of the acid form of gacyclidine, *e.g*., the chloride salt, can be mixed with sufficient base, such as sodium carbonate, to convert it to the basic form. This can be done in solution to form an aqueous suspension of gacyclidine base, which is then formulated into the gel or the pH is adjusted to the acid form already formulated into a gel to produce *in situ* the free base inside the gel. Similarly the free base can be prepared *in situ* within a collagen sponge following impregnation with the acidic form of gacyclidine, and then adjusting the pH with a suitable base to produce the desired sponge loaded with internalized, adsorbed gacyclidine base.

Alternatively, because the basic form of gacyclidine is soluble in organic solvents, such as ethanol, isopropanol, acetone, methylene chloride or dimethyl sulfoxide, solutions of the base form of gacyclidine dissolved in an organic solvent can be applied to a collagen sponge to impregnate the sponge with the base form of gacyclidine leaving behind solid drug after evaporation of the solvent

### Nanoparticles

Nanoparticles are particularly useful as solid carriers. Nanoparticle formulations can pass through an antibacterial filter prior to injection into the cochlea to ensure sterility. *See, e.g.,* Prakobvaitayakit et al., AAPS PharmaSciTech 4(4), E71, 2003; U. S. Patent 6,139,870.

### 1. Materials

Both biodegradable and non-degradable materials can be used to form nanoparticles. See Orive et al. Cancer Therapy 3, 131-38, 2005; Lu et al. Adv Drug Deliv Rev 56, 1621-33, 2004. Bioerodible polymers for nanoparticle formulation can be prepared from lactic and glycolic acid. Such polymers are commercially available (e.g., from Lakeshore Biomaterials, Birmingham, AL) and have measured rates of biodegradation ranging from 2-3 weeks to 12-16 months. Such polymers can be employed to prepare nanoparticles that will release drug within a known period of time.

### 2. Nanoparticle Fabrication Methods

A variety of methods can be employed to fabricate nanoparticles of suitable size *(e.g.,* 10-1000 nm). These methods include vaporization methods, such as free jet expansion, laser vaporization, spark erosion, electro explosion and chemical vapor deposition; physical methods involving mechanical attrition (e.g., "pearlmilling" technology, Elan Nanosystems), super critical CO₂ and interfacial deposition following solvent displacement. The solvent displacement method has the advantage of being relatively simple to implement. The size of nanoparticles produced by this method is sensitive to the concentration of polymer in the organic solvent; the rate of mixing; and to the surfactant employed in the process.

Natural surfactants, such as cholic acid or taurocholic acid salts, can be employed to prepare small nanoparticles (< 100 nm) or to be used as solubilizing and stabilizing excipients in the formulation. Taurocholic acid, the conjugate formed from cholic acid and taurine, is a fully metabolizable sulfonic acid surfactant. An analog of taurocholic acid, tauroursodeoxycholic acid (TUDCA), is also known to have neuroprotective and anti-apoptotic properties; see Rodrigues et al. Proc Natl Acad Sci USA 100, 6087-92, 2003. TUDCA is a naturally occurring bile acid and is a conjugate of taurine and ursodeoxycholic acid (UDCA). Other naturally occurring anionic (e.g., galactocerebroside sulfate), neutral (e.g., lactosylceramide) or zwitterionic surfactants (e.g., sphingomyelin, phosphatidyl choline, palmitoyl carnitine) could also be used in the method of the invention to prepare nanoparticles or as solubilizing and chemically stabilizing excipients.

### 3. Mixing technology

When preparing nanoparticles by the solvent displacement method, a stirring rate of 500 rpm or greater is considered optimum. Slower solvent exchange rates during mixing produce larger particles. Continuous flow mixers can provide the necessary turbulence to ensure small particle size. One type of continuous flow mixing device that can be used in the method of the invention to prepare small nanoparticles (< 100 nm) is known as a Wiskind mixer; see Hansen et al. J Phys Chem 92, 2189-96, 1988. In other embodiments sonilators or sonicators and other kinds of ultra sonic devices; flow through homogenizers such as Gaulin-type homogenizers or super critical CO₂ devices may be used to prepare nanoparticles.

### 4. Particle sizing

Size-exclusion chromatography (SEC), one embodiment of which is known as gelfiltration chromatography, can be used to separate particle-bound from free drug or to select a suitable size range of drug-containing nanoparticles. Various SEC media with molecular weight cut-offs (MWCO) for globular proteins ranging from 200,000 (Superdex 200); to about 1,000,000 (Superose 6); to greater than 10⁸ (Sephacryl 1000; suitable for SEC separation of viruses and small particles > 1 µm) are commercially available (e.g., from GE Healthcare, Amersham Biosciences, Uppsala, Sweden). If suitable nanoparticle homogeneity is not obtained on direct mixing, then SEC can be used to produce highly uniform drug-containing particles that are freed of other components (e.g., solvents and surfactants) involved in their fabrication. Particles can be separated by size by cyclones, centrifugation, membrane filtration and by use of other molecular sieving devices, crosslinked gels/materials and membranes. Other kinds of solid particles include but are not limited to prepared particles, such as TECHNOSPHERE^{®}s which can be formulated after the particle is formulated. TECHNOSPHERE^{®}s can be coated by the therapeutic or prepared as drug-carrier homogeneous particles. Such particles can be selected to be insoluble in acidic conditions and soluble at neutral/basic conditions or vice versa depending on if the DKP has acidic or basic side chains.

The particles can be comprised of soluble, erodible or non-erodible materials depending on the circumstances. As one embodiment the basic form of gacyclidine can be formulated within a homogeneous silastic or silicone polymer material and released following diffusion through the material to the surface in contact with the physiological fluids. This drug-containing polymeric material can be used in a stand alone delivery device or coated onto another device. In another embodiment solid basic form of the drug can be formulated into particles which have a coating that delays or slows the release of the drug. In other embodiments there can be a core reservoir of concentrated drug using a membrane to modulate release of the drug.

### Thin film compositions ofgacyclidine and other therapeutics

Therapeutic agents such as the basic form of gacyclidine may be formulated as "entrapped" compounds within polymeric matrices or layers either as soluble solutions or "embedded" as insoluble heterogeneous aggregates/crystals within polymeric matrices. Examples of polymeric matrices include, but are not limited to, silastic or silicone rubber, polyurethane and polyvinyl chloride. The therapeutic agents will be released from the polymeric coating by diffusion, stimulated release (via electric charge; electrophoresis), mechanical pressure (coating compression to extrude the agent via channels) or erosion to reveal therapeutic agents that are not able to diffuse to the polymeric surface.

In other embodiments, a multilayer film contains an inner layer containing pure drug or a concentrated drug/matrix layer covered with a membrane or outer layer that will modulate the release of the drug coming from the inner layer. The desirable release rate will be established after considering a variety of conditions determined by the nature of the therapeutic, the length of time needed for therapeutic effect, desired release rate profile. The outer layer can be an erodible polymer so that initially there will not be therapeutic release until the outer layer is eroded.

Pore size of the coatings will determine the release rate by the amount of cross linking of the polymer coatings. The optimum pore size will be coordinated to the kind of material being delivered and its requirements. In some embodiments pH and polymer film ion composition can be varied to allow induction of membrane pore changes to accommodate different diffusion rates when presented with different environmental pH's or different electrical charges induced by the management of the film by the device, for example, the electrode array of a cochlear implant.

In some embodiments the inner most layer is refilled through a specially designed port to recharge the lower layer and provide for a longer period of release. Polymeric coatings of this kind are used to provide a pseudo zero order release rate for weeks and months depending on the therapeutic until the drug is exhausted. These coatings can be used for chronic conditions if they have a refillable reservoir. Coatings that cannot be refilled are typically used for acute conditions.

In other embodiments, the thin film contains and delivers multiple therapeutics simultaneously for various purposes. For example, multiple therapeutics can be used to simultaneously inhibit infection and inflammation as well as tinnitus following implantation of a cochlear implant.

In other embodiments, a thin film is layered onto an electro-stimulation device and contains a charged therapeutic, such as the acid form of gacyclidine. Elution of the therapeutic can then be accelerated or diminished based on the charge of the electrode.

### Other therapeutic agents

This invention makes practical the formulation and administration to the cochlea, inner ear, middle ear, auditory cortex, inferior colliculus or other appropriate site of therapeutic agents which have little solubility under physiologically acceptable conditions either alone or in combination with gacyclidine depending on the application and method of delivery. For example, other potent CNS drugs may be co-formulated to treat vestibular disorders together with tinnitus. For example, drugs which inhibit inflammation, an immune response, or fibrosis may be beneficial to combine with the tinnitus therapeutic. Antibiotic drugs also may be co-formulated or mixed with the tinnitus therapeutic to gain the simultaneous benefit of preventing or treating infections with tinnitus treatment. The delivery of multiple agents separately to the middle or inner ear is difficult on the patient because of the invasive nature of the process. Combination therapies offer benefits to the patient by treating one or more disease mechanisms simultaneously while improving the convenience to the physician. Examples of multiple agents that can be used in combination with gacyclidine are listed below.

These therapeutic agents include but are not limited to those useful for the restoration of hearing (e.g., neurotrophins and other growth factors and proteins useful for promoting hearing restoration), DNA, RNA, RNAi, siRNA, retinoblastoma protein and other members of the pocket family of proteins, genetic therapy plasmids with useful genes incorporated, antifibrotics to reduce the threshold of hearing in the cochlea, cell cycle inhibitor antagonists for induction of hair cell division and prostaglandins, for example but not limited to misoprostal or latanoprost and so forth. Other therapeutic agents include antibiotics, drugs for the treatment of cancer, antibacterial agents, antiviral agents, anti-inflammatory agents such as steroids including but not limiting to methyl prednisolone, dexamethasone, triamcinolone acetonide, antioxidants (including but not limited to agents that neutralize or prevent radical and superoxide species; superoxide dismutase mimetics; peroxidases or peroxididase mimetics such as ebselen; reducing agents such as disulfide species) and antiapoptotic agents (such as but not limited to caspases including caspase 3, 9 and/or 12 inhibitors which are believed to prevent hair cell death) to prevent noise, trauma and age dependent hearing loss. These formulations also are especially suitable for the administration of NMDA receptor antagonists to the cochlea, inner ear, or middle ear either alone or in combination with one or more of these kinds of agents in a single formulation.

Therapeutic compounds which can be used to treat middle and inner ear disorders according to the invention include those currently marketed as anxiolytics, antidepressants, selective serotonin reuptake inhibitors (SSRI), calcium channel blockers, sodium channel blockers, anti-migraine agents (e.g., flunarizine), muscle relaxants, hypnotics, and anti-convulsants, including anti-epileptic agents. Examples of such compounds are provided below.

### Anticonvulsants

Anticonvulsants include barbiturates (e.g., mephobarbital and sodium pentobarbital); benzodiazepines, such as alprazolam (XANAX^{®}), lorazepam, clonazepam, clorazepate dipotassium, and diazepam (VALIUM^{®}); GABA analogs, such as tiagabine, gabapentin (an α2δ antagonist, NEURONTIN^{®}), and β-hydroxypropionic acid; hydantoins, such as 5,5-diphenyl-2,4-imidazolidinedione (phenytoin, DILANTIN^{®}) and fosphenytoin sodium; phenyltriazines, such as lamotrigine; succinimides, such as methsuximide and ethosuximide; 5H-dibenzazepine-5-carboxamide (carbamazepine); oxcarbazepine; divalproex sodium; felbamate, levetiracetam, primidone; zonisamide; topiramate; and sodium valproate.

### NMDA receptor antagonists

There are many known inhibitors of NMDA receptors, which fall into five general classes. Each of the compounds described below includes within its scope active metabolites, analogs, derivatives, compounds made in a structure analog series (SAR), and geometric or optical isomers which have similar therapeutic actions.

### Competitors for the NMDA receptor glutamate binding site

Antagonists which compete for the NMDA receptor's glutamate-binding site include LY 274614 (decahydro-6-(phosphonomethyl)-3-isoquinolinecarboxylic acid), LY 235959 [(3S,4aR,6S,8aR)-decahydro-6-(phosphonomethyl)-3-isoquinolinecarboxylic acid], LY 233053 ((2R,4S)-rel-4-(1H-tetrazol-5-yl-methyl)-2-piperidine carboxylic acid), NPC 12626 (α-amino-2-(2-phosphonoethyl)-cyclohexanepropanoic acid), reduced and oxidized glutathione, carbamathione, AP-5 (5-phosphono-norvaline), CPP (4-(3-phosphonopropyl)-2-piperazine-carboxylic acid), CGS-19755 (seifotel, cis-4(phono-methyl)-2-piperidine-carboxylic acid), CGP-37849 ((3E)-2-amino-4-methyl-5-phosphono-3-pentenoic acid), CGP 39551 ((3E)-2-amino-4-methyl-5-phosphono-3-pentenoic acid, 1-ethyl ester), SDZ 220-581 [(αS)-α-amino-2'-chloro-5-(phosphonomethyl)-[1,1'-biphenyl]-3-propanoic acid], and S-nitrosoglutathione. See Gordon *et al.,* 2001; Ginski and Witkin, 1994; Calabresi *et al.,* 2003; Hermann *et al.,* 2000; Kopke *et al.,* 2002; Ikonomidou and Turski, 2002; Danysz and Parsons, 1998.

### Non-competitive inhibitors which act at the NMDA receptor-linked ion channel

Antagonists which are noncompetitive or uncompetitive and act at the receptor-linked ion channel include amantadine, aptiganel (CERESTAT^{®}, CNS 1102), caroverine, dextrorphan, dextromethorphan, fullerenes, ibogaine, ketamine, lidocaine, memantine, dizocilpine (MK-801), neramexane (MRZ 2/579, 1,3,3,5,5-pentamethyl-cyclohexanamine), NPS 1506 (delucemine, 3-fluoro-γ-(3-fluorophenyl)-N-methyl-benzenepropanamine hydrochloride), phencyclidine, tiletamine and remacemide. See Palmer, 2001; Hewitt, 2000; Parsons *et al.,* 1995; Seidman and Van De Water, 2003; Danysz *et al.,* 1994; Ikonomidou and Turski, 2002; Feldblum *et al.,* 2000; Kohl and Dannhardt, 2001; Mueller *et al.,* 1999; Sugimoto *et al.,* 2003; Popik *et al.,* 1994; Hesselink et al., 1999.

### Antagonists which act at or near the NMDA receptor's polyamine-binding site

Antagonists which are thought to act at or near the NMDA receptor's polyamine-binding site include acamprosate, arcaine, conantokin-G, eliprodil (SL 82-0715), haloperidol, ifenprodil, traxoprodil (CP-101,606), and Ro 25-6981 [(±)-(R,S)-α-(4-hydroxyphenyl)-β-methyl-4-(phenylmethyl)-1-piperidine propanol]. See Mayer *et al.,* 2002; Kohl and Dannhardt, 2001; Ikonomidou and Turski, 2002; Lynch *et al.,* 2001; Gallagher et *al.,* 1996; Zhou *et al.,* 1996; 1999; Lynch and Gallagher, 1996; Nankai et al., 1995.

### Antagonists which act at the NMDA receptor's glycine-binding site

Antagonists which are thought to act at the receptor's glycine-binding site include aminocyclopropanecarboxylic acid (ACPC), 7-chlorokynurenic acid, D-cycloserine, gavestinel (GV-150526), GV-196771A (4,6-dichloro-3-[(E)-(2-oxo-1-phenyl-3-pyrrolidinylidene)methyl]-1H-indole-2-carboxylic acid monosodium salt), licostinel (ACEA 1021), MRZ-2/576 (8-chloro-2,3-dihydropyridazino[4,5-b]quinoline-1,4-dione 5-oxide 2-hydroxy-N,N,N-trimethyl-ethanaminium salt), L-701,324 (7-chloro-4-hydroxy-3-(3-phenoxyphenyl)-2(1H)-quinolinone), HA-966 (3-amino-1-hydroxy-2-pyrrolidinone), and ZD-9379 (7-chloro-4-hydroxy-2-(4-methoxy-2-methylphenyl)-1,2,5,10-tetra-hydropyridanizo[4,5-b]quinoline-1,10-dione, sodium salt). Peterson *et al.,* 2004; Danysz and Parsons, 2002; Ginski and Witkin, 1994; Petty el al., 2004; Danysz and Parsons, 1998.

### Antagonists which act at the NMDA receptor's allosteric redox modulatory site

Antagonists which are thought to act at the allosteric redox modulatory site include oxidized and reduced glutathione, S-nitrosoglutathione, sodium nitroprusside, ebselen, and disulfiram (through the action of its metabolites DETC-MeSO and carbamathione). See Hermann *et al.,* 2000; Ogita *et al.,* 1998; Herin *et al.,* 2001, Ningaraj *et al.,* 2001; Kopke *et al.,* 2002.

Some NMDA receptor antagonists, notably glutathione and its analogs (S-nitrosoglutathione and carbamathione), can interact with more than one site on the receptor.

CNQX (1,2,3,4-tetrahydro-7-nitro-2,3-dioxo-6-quinoxalinecarbonitrile) and DNQX (1,4-dihydro-6,7-dinitro-2,3-quinoxalinedione) bind to non-NMDA glutamate receptors. These and other antagonists or agonists for glutamate receptors can be used in the methods of the invention.

It is preferable that the NMDA receptor antagonists, like those disclosed herein, inhibit NMDA receptors without inhibiting AMPA receptors. The reason for this is that inhibition of AMPA receptors is thought to result in impairment of hearing. By contrast, selective inhibition of NMDA receptors is expected to prevent initiation of apoptosis, programmed cell death, of the neuron. Unlike AMPA receptors, which are activated by glutamate alone, NMDA receptors require a co-agonist in addition to glutamate. The physiologic co-agonist for NMDA receptors is glycine or D-serine. NMDA receptors but not AMPA receptors also bind reduced glutathione, oxidized glutathione, and S-nitrosoglutathione. Glutathione, γ-glutamyl-cysteinyl-glycine, is thought to bridge between the glutamate and glycine binding sites of NMDA receptors, binding concurrently at both sites. Activation of NMDA receptors leads to entry of calcium ions into the neuron through the linked ion channel and initiation of Ca²⁺-induced apoptosis. Intracellular calcium activates the NMDA receptor-associated neuronal form of nitric oxide synthase (nNOS), calpain, caspases and other systems linked to oxidative cell damage. Inhibition of NMDA receptors should prevent death of the neuron.

### Subtype-specific NMDA receptor antagonists

A variety of subtype-specific NMDA receptor agonists are known and can be used in methods of the invention. For example, some NMDA receptor antagonists, such as arcaine, argiotoxin636, Co 101244 (PD 174494, Ro 63-1908, 1-[2-(4-hydroxyphenoxy)ethyl]-4-[(4-methylphenyl)methyl-4-piperidinol], despiramine, dextromethorphan, dextrorphan, eliprodil, haloperidol, ifenprodil, memantine, philanthotoxin343, Ro-25-6981 ([(±)-(R*, S*)-α-(4-hydroxyphenyl)-β-methyl-4-(phenylmethyl)-1-piperidine propanol]), traxoprodil (CP-101,606), Ro 04-5595 (1-[2-(4-chlorophenyl)ethyl]-1,2,3,4-tetrahydro-6-methoxy-2-methyl-7-isoquinolinol), CPP [4-(3-phosphonopropyl)-2-piperazinecarboxylic acid], conantokin G, spermine, and spermidine have moderate or high selectivity for the NR2B (NR1A/2B) subtype of the receptor. NVP-AAM077 [[[[(1S)-1-(4-bromophenyl)ethyl]amino](1,2,3,4-tetrahydro-2,3-dioxo-5-quinoxalinyl)methyl]-phosphonic acid] is an NR2A subtype-specific antagonist. See Nankai *et al,* 1995; Gallagher *et al.,* 1996; Lynch and Gallagher, 1996; Lynch *et al,* 2001; Zhou *et al.,* 1996; Zhou *et al.,* 1999; Kohl and Dannhardt, 2001, Danysz and Parsons, 2002.

Antagonists such as 1-(phenanthrene-2-carbonyl) piperazine-2,3-dicarboxylic acid (Feng et al., Br J Pharmacol 141, 508-16, 2004), which has high selectivity for the NR2D and 2C subtypes of the receptor, are particularly useful.

### Useful therapeutics other than NMDA receptor antagonists

Other useful therapeutic agents which can be formulated and administered according to the invention include nortriptyline, amytriptyline, fluoxetine (PROZAC^{®}), paroxetine HCl (PAXIL^{®}), trimipramine, oxcarbazepine (TRILEPTAL^{®}), eperisone, misoprostol (a prostaglandin E₁ analog), latanoprost (a prostaglandin F₂α-analog) melatonin, and steroids (e.g., pregnenolone, triamcinolone acetonide, methylprednisolone, and other anti-inflammatory steroids).

N-type calcium channel blockers, such as Piralt (Takizawa et al., Cereb Blood Flow Metab 15, 611-8, 1995) also can be formulated and administered according to the invention.

### Therapeutic methods

Gacyclidine formulations as described above are useful to treat mammals (including livestock and companion animals) and particularly humans. They are especially useful for treating tinnitus, including tinnitus associated with neurological disorders, such as Meniere's Disease, pain, anxiety, depression, and migraine headaches.

Formulations of the invention can be administered by various methods. For example, a round window catheter (*e*.*g*., U. S. Patents 6,440,102 and 6,648,873) can be used.

Alternatively, formulations can be incorporated into a wick for use between the outer and middle ear (*e*.*g*., U. S. Patent 6,120,484) or absorbed to collagen sponge or other solid support (e.g., U. S. Patent 4,164,559).

If desired, formulations of the invention can be incorporated into a gel formulation (e.g., U. S. Patents 4,474,752 and 6,911,211). Formulations of the invention also can be administered by direct injection into the middle ear, inner ear, or cochlea (e.g., U. S. Patent 6,377,849 and Serial No. 11/337,815).

Formulations of the invention also can be delivered via an implanted pump and delivery system through a needle directly into the middle or inner ear (cochlea) or through a cochlear implant stylet electrode channel or alternative prepared drug delivery channel such as but not limited to a needle through temporal bone into the cochlea.

Other options include delivery via a pump through a thin film coated onto a multichannel electrode or electrode with a specially imbedded drug delivery channel (pathways) carved into the thin film for this purpose. In other embodiments the acidic or basic solid gacyclidine can be delivered from the reservoir of an external or internal implanted pumping system.

### Delivery of acidic or basic formulations of gacyclidine through implanted drug delivery systems

For long term delivery to tinnitus patients a convenient method of delivery is needed such as an implanted drug delivery system. For use in such a system, the drug can be formulated as a homogeneous acidic or basic form of gacyclidine or a mixture of the acidic and basic forms with the appropriate excipients to ensure chemical stability and adequate solution concentrations.

Formulations of the invention also can be delivered via elution from a drug loaded thin film or reservoir on the surface of an electrode, contained within the electrode polymeric matrix or other delivery material implanted in the middle or inner ear. Gacyclidine can be delivered from a drug loaded electrode via an electrophoresis method following the charging of the electrode to drive the movement of the charged gacyclidine.

Gacyclidine base can also be pressed or cast into various solid shapes, such as but not limited to tablets, which can be implanted or subjected to slow erosion by a moving liquid stream. Such solid shapes, as well as vehicle suspended nanoparticles, are then eroded by solutions from the delivery system's reservoir or with fluids withdrawn from the patient and returned saturated with drug to the patient.

All patents, patent applications, and references cited in this disclosure are expressly incorporated herein by reference. The above disclosure generally describes the present invention. A more complete understanding can be obtained by reference to the following specific examples, which are provided for purposes of illustration only and are not intended to limit the scope of the invention.

### EXAMPLE 1

### Method of assessing chemical stability and solubility of gacyclidine

This example describes analytical methods that can be used to determine chemical stability and the solution concentration of gacyclidine in various formulations.

### Method A (rapid; determination of solution concentration of gacyclidine only)

Gacyclidine was detected using a Surveyor HPLC system (Thermo Electron). A Grace Vydac C8 MASS SPEC column (cat # 208MS5210; S/N NE981208-3-7) purchased from The Nest Group (Southboro, MA) was employed for these analyses. The chromatographic column was maintained at 30°C. Samples were prepared in 12 mm x 32 mm autosampler vials (Thermo Electron, A4954-010) and maintained at 20°C in the autosampler.

For maximum accuracy, a 'full-loop' injection protocol was employed, in which 80.7 µL was withdrawn from the vial to over-fill a 20 µL injection loop. The entire sample contained in the 20 µL injection loop was applied to the C8 chromatography column. The sample was then eluted at a flow rate of 300 µL/min with a step gradient composed of water containing 0.1% (volume/volume) trifluoroacetic acid (buffer A) and acetonitrile containing 0.1% (volume/volume) trifluoroacetic acid (buffer B). The step gradient consisted of elution with 100% buffer A from 0 to 5 minutes; 80% buffer A-20% buffer B from 5 to 15 minutes; 100% buffer B from 15 to 20 minutes and 100% buffer A from 20 to 25 minutes.

The column eluant was monitored at 234 nm by use of a Thermo Electron PDA UV detector. Gacyclidine eluted from the column at 10.0 minutes and could easily be detected at a solution concentration of 1 µM. The presence of gacyclidine was confirmed by connecting the column eluant, after passage through the UV detector, to an AQA mass spectrometer (Thermo Electron). Gacyclidine was detected at the appropriate elution position by use of the mass spectrometer in the positive ion mode with selective ion monitoring (SIM) at a mass to charge ratio (m/z) of 264.

### Method B (determination of chemical stability: simultaneous determination of both gacyclidine and piperidine)

To monitor piperidine formation a Zorbax SB-CN column (25 x 0.46 cm, 5 µm; PN 880975, SN USS F01 3866) purchased from Agilent Technologies was maintained at 30°C and eluted with a mixture composed of 50 % Buffer A and 50 % Buffer B at 150 µL/minute for 60 minutes. Buffer A was composed of water containing 0.1 % trifluoroacetic acid and Buffer B was composed of acetonitrile containing 0.1 % trifluoroacetic acid. The effluent from this column was interfaced with an AQA single quadrupole mass spectrometer (Thermo Electron) and a Surveyor PDA detector (UV monitor, Thermo Electron). The column effluent was monitored at 232 nm and scans were collected between 200 to 300 nm by use of the PDA detector. Gacyclidine was detected by the PDA detector at 28.1 minutes after injection onto the Zorbax SB-CN column. The column effluent was also monitored by SIM (selective ion monitoring) at a mass-to-charge (m/z) ratio of 264.46 (m+1 for gacyclidine) and 86.16 (m+1 for piperidine) by use of the AQA mass spectrometer. Gacyclidine was detected by the mass spectrometer at 28.3 minutes after injection onto the Zorbax SB-CN column (at m/z = 264 and 86). Piperidine was detected by the mass spectrometer at 16.0 minutes after injection onto the Zorbax SB-CN column (at m/z = 86 only). Piperidine could not be detected by use of the PDA (UV monitor).

### EXAMPLE 2

### Maintenance of gacyclidine in solution

Stock solutions (0.1 M) of gacyclidine (gacyclidine hydrochloride salt) were prepared in either dimethyl sulfoxide (DMSO) or water. An aliquot (10 µL) of these solutions was then added to 10.0 mL of either Ringer's Lactate (pH 6.6), Ringer's Lactate containing 100 µM NaOH (pH 7.5), or 0.1 N HCl (pH 1.0) to give a final drug concentration of 100 µM. Aliquots of these drug solutions were transferred to autosampler vials and maintained at 20°C until being analyzed by HPLC. The results are summarized in Table 2.

**Table 2. Concentration of gacyclidine in various formulations over time**

| DMSO drug stock diluted 1:1000 (0.1% final concentration) | | | |
|---|---|---|---|
| | 0-3 hours | 3-6 hours | 16-19 hours |
| Ringer's Lactate, pH 7.5 | 97.5 µM | 90.4 µM | 79.3 µM |
| Ringer's Lactate, pH 6.6 | 96.0 µM | 94.9 µM | 91.3 µM |
| 0.1 N HCl | 100.3 µM | 100.2 µM | 100.6 µM |
| | | | |

| Aqueous drug stock diluted 1:1000 | | | |
|---|---|---|---|
| Ringer's Lactate, pH 7.5 | 88.2 µM | 72.0 µM | 59.7 µM |
| Ringer's Lactate, pH 6.6 | 95.1 µM | 92.6 µM | 87.7 µM |
| 0.1 N HCl | 99.4 µM | 99.8 µM | 100.2 µM |

As can be seen in Table 2, solutions of gacyclidine under physiologic conditions (for example, dissolved in Ringer's Lactate solution, pH 6.6, or in Ringer's Lactate which has had its pH adjusted to 7.5) do not maintain gacyclidine at the initial concentrations used. By contrast, gacyclidine dissolved under acidic conditions (0.1 N HCl which is pH 1.0 in Table 2) is maintained in solution for several days at room temperature. Addition of acid to vials which apparently have reduced concentrations of gacyclidine in solution increases the detectable amount of gacyclidine. This indicates that the reductions in gacyclidine concentration over time documented in Table 2 are predominantly due to the compound coming out of solution.

As documented in Table 2, 0.1% DMSO retards the loss of gacyclidine from solution. Higher concentrations of DMSO, such as 0.1-100%, can further improve the ability of gacyclidine to stay in solution.

### EXAMPLE 3

### Solubility ofgacyclidine

The hydrochloride salt of gacyclidine produced a clear solution almost immediately on contact with water, up to a final concentration of 550 mM. However, if a 100 mM solution of the hydrochloride salt was diluted into a buffered aqueous solution to a final concentration of 1 mM, then precipitation of gacyclidine was observed above pH 7.

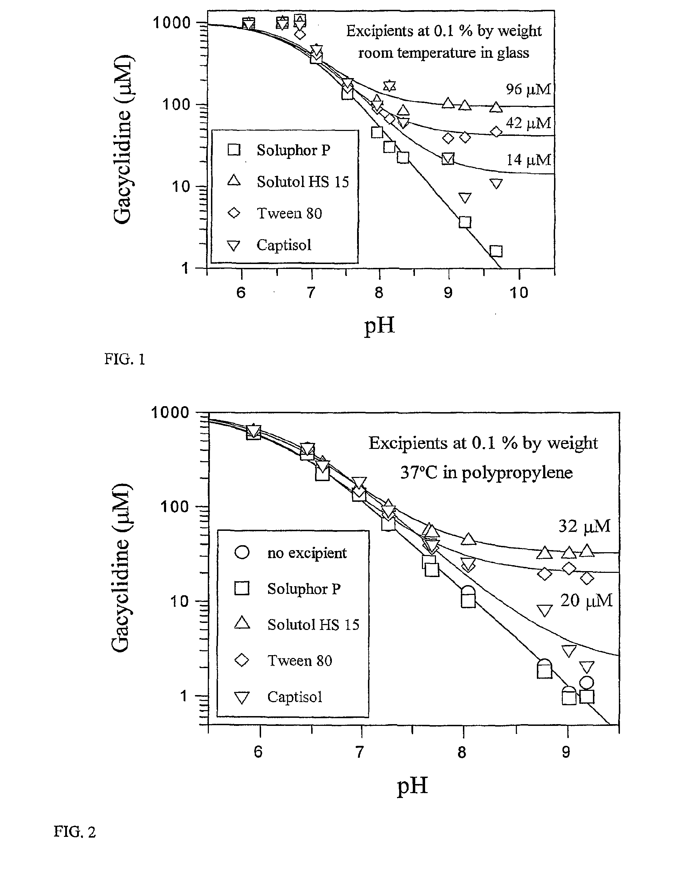

Figure 1 shows the amount of gacyclidine that remained in solution after 10 µL of a 100 mM solution of the hydrochloride salt was added to 0.99 mL of a buffered solution. Thereafter the mixture remained standing at room temperature for two days. The buffered solutions contained 100 mM sodium chloride and one of the following buffering substances at 50 mM concentration at a pH equivalent to the buffer's pKa (*i*.*e*., the buffer was 50% in its acidic form and 50% in its basic form): MES, Bis-Tris, MOPSO, MOPS, TAPSO, Tris, Tricine, TAPS, CHES, AMPSO, or CAPSO (Sigma Chemical Company, St. Louis, MO).

The pH of the mixture was determined by use of a SympHony glass calomel micro combination pH electrode obtained from VWR (Cat. No. 14002-776). Oakton standard reference solutions at pH 7.00 ± 0.01 (25°C; 7.02 at 20°C), 4.01 ± 0.01 (25°C; 4.00 at 20°C) and 10.00 (25°C; 10.05 at 20°C) were obtained from Cole-Parmer. These standard pH solutions were used to calibrate a Cole-Parmer Model 5996-60 analog pH meter equipped with a SympHony electrode to within 0.01 pH units of reference solutions.

When the pH of the mixture was higher than pH 7, gacyclidine precipitated from solution and settled to the bottom of glass autosampler vials (Thermo Electron, A4954-010) as a visible white precipitate of gacyclidine base within 24 hours. Including 0.1 % SOLUPHOR^{®} P (BASF) in the mixture did not increase the solubility of gacyclidine above pH 7. At pH 9.7 the amount of gacyclidine remaining in solution after standing at room temperature for two days was only 2.6 µM, 0.26 % of the added gacyclidine, in the presence (Figure 1) or absence (not shown) of SOLUPHOR^{®} P. By contrast, including 0.1 % CAPTISOL^{®}, TWEEN^{®} 80, or SOLUTOL^{®} HS 15 in the mixture increased the amount of gacyclidine remaining in solution at high pH to about 14, 42 or 96 µM (1.4, 4.2, or 9.6 % of the gacyclidine added), respectively.

This same procedure was conducted at 37°C, and the samples were maintained in polypropylene vials (VWR # 20170-710, 2 mL vial). The results are shown in Figure 2. Loss of gacyclidine from solution was observed even at pH 6 and by pH 9.2 the concentration of gacyclidine remaining in solution in the presence or absence of 0.1 % SOLUPHOR^{®} P was only about 1 µM (0.1 % of the total gacyclidine added). As before, addition of 0.1 % TWEEN^{®} 80 or SOLUTOL^{®} HS 15 to the mixture increased the amount of gacyclidine retained in solution at high pH to a concentration of about 20 or 32 µM (2.0 or 3.2 % of total gacyclidine added, respectively). Addition of 0.1 % CAPTISOL^{®} to the mixture at 37°C in polypropylene containers did not result in a clearly defined solubility for the basic form of gacyclidine (no plateau).

Because polypropylene reduced the amount of 100 µM gacyclidine remaining in solution, the ability of SOLUTOL^{®} HS 15 to retain 25 µM gacyclidine in the absence of excess precipitated gacyclidine was tested. Solutions containing 25 µM gacyclidine were prepared in 0.15 M sodium chloride and the pH of these solutions was adjusted by addition of sodium bicarbonate or hydrochloric acid. Some of these solutions also contained 0.1 % SOLUTOL^{®} HS 15. After incubating at 37°C in polypropylene vials for one or three days, the concentration of gacyclidine remaining in these solutions was determined.

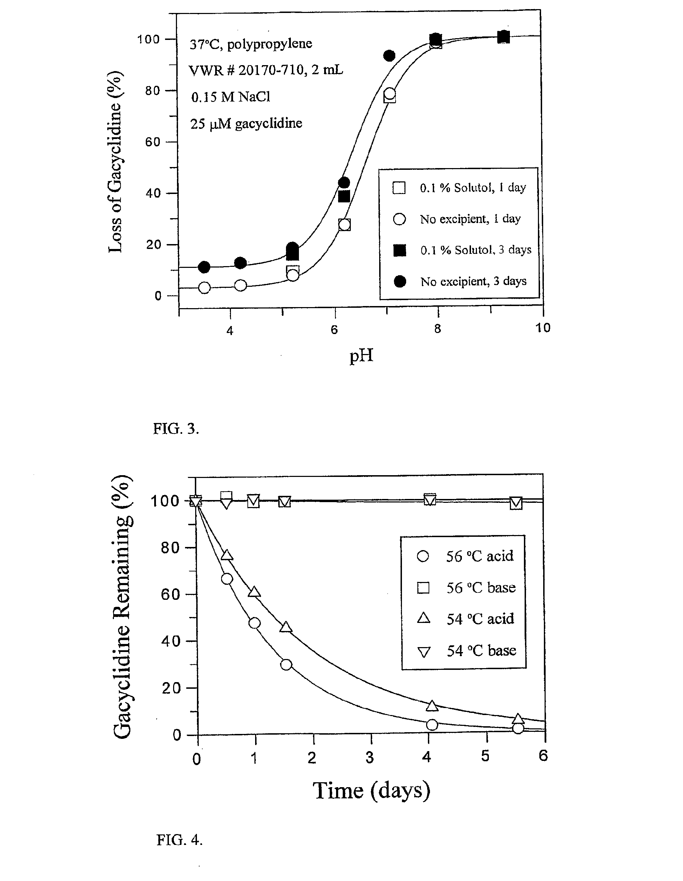

Figure 3 shows the loss of gacyclidine from solution as a function of pH. Above pH 6, losses of gacyclidine were observed and the amount of loss increased with an increase in pH. Although the results after three days of incubation parallel those obtained after one day of incubation at 37°C, the increased loss observed at low pH in the samples incubated for three days presumably reflected losses due to decomposition. Although 0.1 % SOLUTOL^{®} HS 15 should have been able to retain 25 µM gacyclidine in solution, based on the results shown in Figure 2, inclusion of 0.1 % SOLUTOL^{®} had no effect on the losses observed under these conditions. These results indicate that in the absence of excess precipitated gacyclidine, SOLUTOL^{®} HS 15 at 0.1 % cannot effectively compete with the polypropylene vial for gacyclidine. Although excipients can solubilize gacyclidine (Figures 1 and 2), other polymeric materials encountered by the solution, such as the polypropylene vial, may have higher affinity for gacyclidine than the excipient.

### EXAMPLE 4

### Chemical stability of gacyclidine

One set of samples was prepared by mixing 0.98 mL of water with 0.01 mL of 100 mM gacyclidine and 0.01 mL of 1.0 M hydrochloric acid in glass autosampler vials. Gacyclidine was completely soluble in this set of samples, and the solution had a pH of 2.0. A second set of samples was prepared by mixing 0.97 mL of water with 0.01 mL of 100 mM gacyclidine and 0.01 mL of 0.15 M sodium hydroxide in glass autosampler vials. Upon mixing, gacyclidine precipitated from solution in these samples and the suspension of gacyclidine base had a pH of 10.4. Both sets of samples were then incubated for various lengths of time at either 54 or 56°C. Following incubation at 54 or 56°C, 0.01 mL of 1.0 M hydrochloric acid was added to the samples containing a suspension of gacyclidine base (0.5 mM excess sodium hydroxide, pH 10.4), which converted gacyclidine to the soluble acid form and converted these suspensions to clear solutions (pH 2.1). After incubation at 54 or 56°C, both sets of samples were analyzed for residual gacyclidine and the formation of piperidine using Method B (Example 1).

As shown in Figure 4, the basic form of gacyclidine was 200-400 times more chemically stable than the acid form. The decomposition rate of 1.0 mM gacyclidine suspended in 1.5 mM sodium hydroxide was 0.0013 day⁻¹ compared to 0.52 day⁻¹ in 10 mM hydrochloric acid at 54°C. Loss of gacyclidine in samples could be completely accounted for by decomposition with the stoichiometric formation of one molecule of piperidine for every molecule of gacyclidine lost.

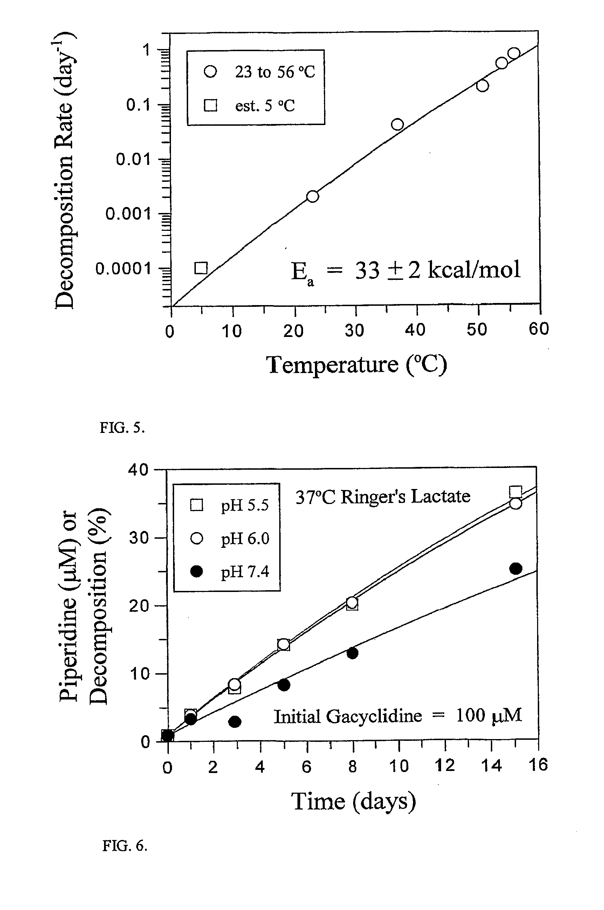

Similar experiments were conducted with the acid form of gacyclidine at a range of incubation temperatures from 5°C to 56°C. The results from these experiments are summarized in Figure 5. The rate of decomposition had a very steep temperature dependence. At 5°C, the rate of decomposition was very slow and was difficult to measure, except by determining piperidine formation. The rate of decomposition at 5°C was approximately 0.0001 day⁻¹, as determined by piperidine formation, or 0.000055 day⁻¹ as determined by extrapolation from the rates at higher temperature according to the Arrhenius relationship. The time required for 10 % loss of gacyclidine (acid form) due to decomposition ranged from 3 years at 5°C, to 53 days at 23°C, to 3.8 days at 37°C, to 3.3 hours at 56°C.

These data confirm the increased cheorical stability of gacyclidine at lower temperatures over body temperature.

To determine the rate of gacyclidine decomposition under near physiologic conditions, 100 µM gacyclidine was prepared in Ringer's Lactate at pH 5.5, 6.0, or 7.4. The pH of these Ringer's Lactate solutions was adjusted by addition of hydrochloric acid or sodium bicarbonate (0.13 mM). A 0.40 mL aliquot of these samples was incubated in acid-washed glass autosampler vials at 37°C for various periods of time and then analyzed for residual gacyclidine and piperidine formation. To analyze these samples for gacyclidine and piperidine, the samples were first extracted with methylene chloride and then back-extracted into dilute hydrochloric acid. To the 0.40 mL sample to be analyzed were added 50 µL of 1.0 M sodium hydroxide and 0.8 mL of methylene chloride. After vigorous mixing, the aqueous and methylene chloride layers were allowed to separate. To 0.4 mL of the methylene chloride phase were added 20 µL of 1.0 M hydrochloric acid and 0.78 mL of water. After vigorous mixing, the aqueous and methylene chloride layers were allowed to separate and the aqueous layer was analyzed for gacyclidine and piperidine. This extraction procedure was necessary to remove the salt present in the sample for optimum detection of piperidine.

As shown in Figure 6, the rates of piperidine formation were nearly equivalent at pH 5.5 and 6.0, but were substantially slower at pH 7.4. Table 3 summarizes the rates obtained by fitting equations for first order decay to data shown in Figure 6. At pH 5.5 or 6.0, the rate of gacyclidine decomposition was equivalent to the rate of the acid form of gacyclidine at 37°C, while the rate at pH 7.4 was only 62 % of the rate expected for the acid form. This would indicate that at pH 7.4 and 37°C only 62 % of gacyclidine is in the acid form and indicate an apparent pK_{A} for gacyclidine of 7.6. This confirms again that the free base of gacyclidine, which predominates in higher pH solutions, is more stable than the acid form, which predominates in lower pH solutions.

**Table 3. Stability of gacyclidine at 37°C.**

| pH | Time for 10 % Loss (37°C, Ringer's Lactate) |
|---|---|
| 5.5 | 3.7 ± 0.1 days |
| 6.0 | 3.82 ± 0.03 days |
| 7.4 | 6.1 ± 0.4 days |

### EXAMPLE 5

### Effect of pH on gacyclidine decomposition and solubility

A series of samples containing 100 µM gacyclidine in Ringer's solution were prepared in acid-washed glass vials. To 10.0 mL of Ringer's solution (Baxter Healthcare Corporation) were added 10 µL of 0.100 M gacyclidine hydrochloride salt and 0.020, 0.040, 0.080, or 0.160 mL of 0.100 M sodium bicarbonate. These samples were then incubated at 55°C for 24 hours. Following incubation at 55°C, a 0.400 mL aliquot was taken from each sample and then analyzed for gacyclidine and piperidine.

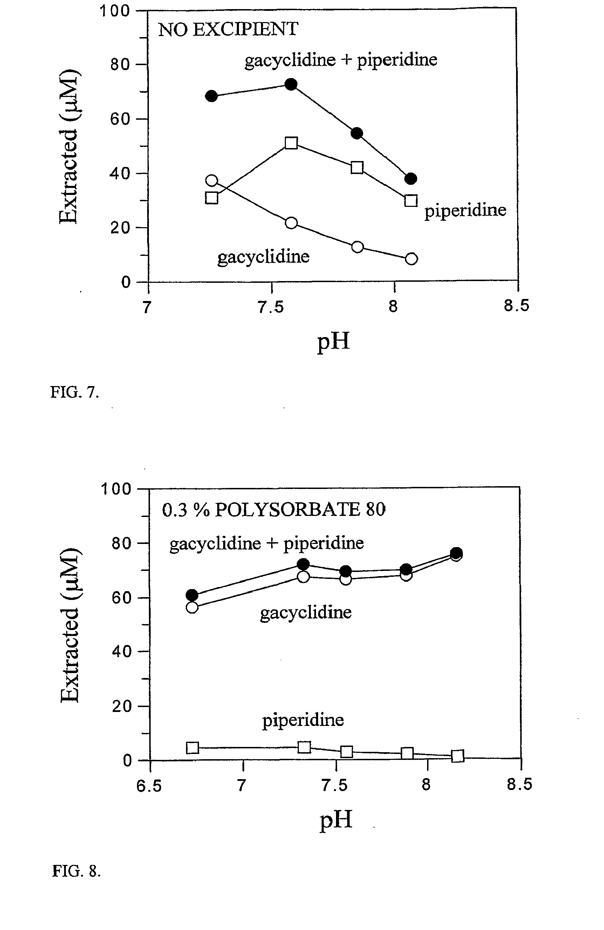

Gacyclidine and piperidine were extracted with methylene chloride and then back-extracted into dilute aqueous acid as described in Example 4. The pH of each sample was determined at 55°C by use of a pH meter standardized at 55°C. The pH meter, electrode and standards were the same as described in Example 3. Figure 7 shows the results obtained for incubation of gacyclidine in Ringer's solution at 55°C at various pH values. In Figure 7, gacyclidine concentration is shown as unfilled circles; piperidine is shown as unfilled squares; and the sum of gacyclidine and piperidine concentrations is shown as filled circles. Clearly, there was loss of gacyclidine both by decomposition and by precipitation from solution as judged by losses of gacyclidine that could not be accounted for by piperidine formation. Precipitation is indicated due to the decrease in the sum of piperidine and gacyclidine concentrations as the pH increases from 7.6 to 8.1. Concomitant with precipitation, the rate of decomposition, as measured by piperidine formation, also decreased from pH 7.6 to 8.1 demonstrating again the increased chemical stability at higher pH.

### EXAMPLE 6

### Effect ofpHandpolysorbate 80 (TWEEN^{®} 80) on gacyclidine stability

A series of samples containing 100 µM gacyclidine and 0.3 % polysorbate 80 (TWEEN^{®} 80) in Ringer's solution were prepared in acid-washed glass vials. To 10.0 mL of Ringer's solution (Baxter Healthcare Corporation) were added 10 µL of 100 mM gacyclidine hydrochloride salt, 0.030 g of polysorbate 80, and 0.02, 0.04, 0.08, 0.16, or 0.32 mL of 100 mM sodium bicarbonate. These samples were then incubated at 55°C for 24 hours. Following incubation at 55°C, a 0.4 mL aliquot was taken from each sample and then analyzed for gacyclidine and piperidine. Gacyclidine and piperidine were extracted with methylene chloride and then back-extracted into dilute aqueous acid as described in Example 4. The pH of each sample was determined at 55°C by use of a pH meter standardized at 55°C. The pH meter, electrode and standards were the same as described in Example 3.

Figure 8 shows the results obtained for incubation of gacyclidine in Ringer's solution containing 0.3 % polysorbate 80 at 55°C and at various pH values. In Figure 8, gacyclidine concentration is shown as unfilled circles; piperidine is shown as unfilled squares; and the sum of gacyclidine and piperidine concentrations is shown as filled circles. There appeared to be no loss of gacyclidine due to precipitation, since the concentration of gacyclidine or the sum of gacyclidine and piperidine appeared to be pH independent. There was also substantially less decomposition of gacyclidine in the presence of 0.3 % polysorbate 80, as measured by piperidine formation. The amount of piperidine formed from 100 µM gacyclidine in the presence of 0.3 % polysorbate 80 varied from 4.5 µM at pH 6.7 to 0.92 µM at pH 8.2. Polysorbate 80 helped to keep gacyclidine in solution, at pH values higher than 7, and dramatically retarded the rate of gacyclidine decomposition, even at a pH lower than 7.

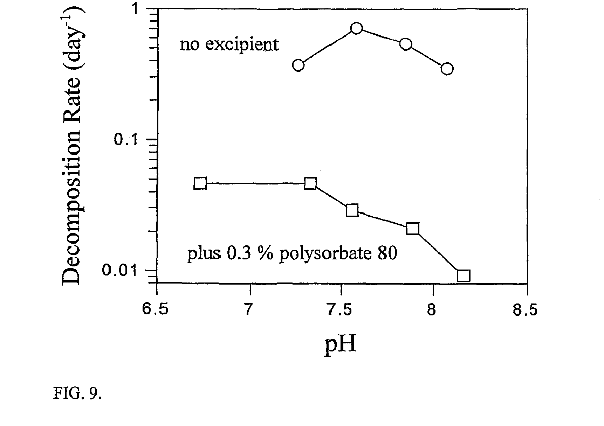

Figure 9 shows a comparison of the rate of gacyclidine decomposition, determined from the concentration of piperidine formed, in the presence and absence of 0.3 % polysorbate 80. The rate of decomposition for the acid form of gacyclidine at 55°C (see Example 4) was 0.73 day⁻¹. The rate for decomposition of gacyclidine in the presence of 0.3 % polysorbate 80 was reduced to 6.4 % of this rate at pH 6.7 and to 1.3 % of this rate at pH 8.2. By contrast, the relative rate for decomposition in the absence of added excipient varied from 51 % of this rate at pH 7.3 to 48 % of this rate at pH 8.1. Polysorbate 80 clearly provided protection against thermal decomposition of gacyclidine, as well as improved solubility of gacyclidine at pH values higher than 7. These results demonstrate that reagents which improve solubility of the basic form of gacyclidine also increase its chemical stability and do so even in a pH range where solubility is not a problem.

### EXAMPLE 7

### Elution of gacyclidine from a suspension of the free base form

This example describes analytical methods that can be used to determine stability of gacyclidine in various formulations.

To determine the solution behavior of suspensions of gacyclidine free base, 2.8 mg of free base powder was suspended in 20.0 mL of Ringer's Lactate, with or without 0.13 mM sodium bicarbonate in a glass vial with continuous stirring. The pH of these solutions was monitored by use of a pH meter, as described in Example 3. Gacyclidine concentrations were determined by HPLC, as described in Example 1. The average temperature of these solutions was 23°C and varied between 21 and 25°C. The results obtained for these solutions are summarized in Table 3.

The initial pH of Ringer's Lactate without added gacyclidine or sodium bicarbonate was 6.7 and increased after the addition of 2.8 mg gacyclidine to a range between 7.4 and 7.7. The concentration of gacyclidine increased within 10 days following addition of the solid to an equilibrium concentration between 152 and 162 µM. The initial pH of Ringer's Lactate containing 0.13 mM sodium bicarbonate was 7.4, near physiological pH, and increased after the addition of 2.8 mg gacyclidine to a range between 7.5 and 7.6. The concentration of gacyclidine increased within 10 days to an equilibrium concentration between 73 and 77 µM.

Based on these results, administration of solid gacyclidine base should result in a slow release of gacyclidine into solution that would take several days to approach a final equilibrium concentration. At a starting pH near physiologic pH, the maximum concentration achieved at equilibrium would be about 75 µM gacyclidine in solution. After two weeks, both solutions still retained the majority of the initial gacyclidine free base added as a suspension of solid. Even after several months, the amount of visible gacyclidine solid appeared to be unchanged. The equilibrium concentrations of gacyclidine achieved by both solutions is only about 30 % or 14 %, respectively, of the total amount of gacyclidine base added to the Ringer's Lactate solution without or with added sodium bicarbonate. These data (Table 4) confirm the suitability of administering solid formulations containing gacyclidine free base.

**Table 4. Elution of gacyclidine from a suspension of solid free base.**

| Time (days) | Ringer's Lactate 133 µM NaHCO₃ | | Ringer's Lactate Without pH Adjustment | |
|---|---|---|---|---|
| | gacyclidine (µM) | pH | gacyclidine (µM) | pH |
| 0 | 0 | 7.43 | 0 | 6.71 |
| 0.625 | 20.7 | 7.54 | 56.4 | 7.66 |
| 1.10 | 37.1 | 7.58 | 79.8 | 7.44 |
| 1.54 | 46.2 ± 0.3 | 7.51 | 88.3 ± 2.4 | 7.46 |
| 5.79 | 76.3 ± 0.3 | 7.62 | 124.4 ± 14.5 | 7.38 |
| 9.85 | 77.2 ± 0.5 | | 152.4 ± 0.7 | |
| 12.9 | 72.9 ± 1.1 | | 162.2 ± 1.4 | |
| 14.0 | 75.7 ± 2.0 | | 157.9 ± 3.6 | |

### REFERENCES

Serial No. 11/337,815
U.S. Patent 4,164,559
U.S. Patent 4,474,752
U.S. Patent 6,107,495
U.S. Patent 6,120,484
U.S. Patent 6,139,870
U.S. Patent 6,377,849
U.S. Patent 6,440,102
U.S. Patent 6,648,873
U.S. Patent 6,911,211
Agerman K, Canlon B, Duan M, Ernfors P (1999) Neurotrophins, NMDA receptors, and nitric oxide in development and protection of the auditory system, Ann NY Acad Sci 884:131-42.
Bugay DE (1999) Pharmaceutical Excipients (Drugs and the Pharmaceutical Sciences, v. 94), Marcel Dekker AG, Basel,
Basile AS, Huang JM, Xie C, Webster D, Berlin C, Skolnick P (1996) N-methyl-D-aspartate antagonists limit aminoglycoside antibiotic-induced hearing loss, Nature Medicine 2:1338-43.
Calabresi P, Centonze D, Cupini LM, Costa C, Pisani F, Bernardi G (2003) Ionotropic glutamate receptors: still a target for neuroprotection in brain ischemia? Insights from in vitro studies, Neurobiol Dis 12:82-8.
Chen Z, Ulfendahl M, Ruan R, Tan L, Duan M (2004) Protection of auditory function against noise trauma with local caroverine administration in guinea pigs, Hear Res 197:131-136.
Chen Z, Ulfendahl M, Ruan R, Tan L, Duan M (2003) Acute treatment of noise trauma with local caroverine application in the guinea pig, Acta Otolaryngol 123:905-909.
Costa E (1998) From GABAA receptor diversity emerges a unified vision of GABAergic inhibition, Ann Rev Pharmacol Toxicol 38:321-350.
Czuczwar SJ, Patsalos PN (2001) The new generation of GABA enhancers. Potential in the treatment of epilepsy, CNS Drugs 15:339-350.
Danysz W, Essmann U, Bresink I, Wilke R (1994) Glutamate antagonists have different effects on spontaneous locomotor activity in rats, Pharmacol Biochem Behav 48:111-8.
Danysz W, Parsons CG (1998) Glycine and N-methyl-D-aspartate receptors: physiological significance and possible therapeutic applications, Pharmacological Reviews 50:597-664.
Danysz W, Parsons CG (2002) Neuroprotective potential of ionotropic glutamate receptor antagonists, Neurotox Res 4:119-26.
Duan M, Agerman K, Ernfors P, Canlon B (2000) Complementary roles of neurotrophin 3 and a N-methyl-D-aspartate antagonist in the protection of noise and aminoglycoside-induced ototoxicity, Proc Natl Acad Sci USA 97:7597-602.
Feldblum S, Arnaud S, Simon M, Rabin O, D'Arbigny P (2000) Efficacy of a new neuroprotective agent, gacyclidine, in a model of rat spinal cord injury, J Neurotrauma 17:1079-93.
Feng B, Tse HW, Skifter DA, Morley R, Jane DE, Monaghan DT (2004) Structure-activity analysis of a novel NR2C/NR2D-preferring NMDA receptor antagonist: 1-(phenanthrene-2-carbonyl) piperazine-2,3-dicarboxylic acid, Br J Pharmacol 141:508-516.
Galici R, Pinna G, Stephens DN, Schneider HH, Turski L (1998) Tolerance to and dependence on Alprazolam are due to changes in GABAA receptor function and are independent of exposure to experimental set-up, Restor Neurol Neurosci 12:233-237.
Gallagher MJ, Huang H, Pritchett DB, Lynch DR (1996) Interactions between ifenprodil and the NR2B subunit of the N-methyl-D-aspartate receptor, J Biol Chem 271:9603-11.
Geneste P, Kamenska JM, Ung SN, Herrmann P, Goudal R, Trouiller G (1979) Determination conformationnelle de dérivés de la phencyclidine en vue d'une correlation structure-activité, Eur J Med Chem 14:301-308.
Gibson M (2004) in Pharmaceutical Preformulation and Formulation: A Practical Guide from Candidate Drug Selection to Commercial Dosage Form, Interpharm/CRC, Boca Raton
Ginski MJ, Witkin JM (1994) Sensitive and rapid behavioral differentiation of N-methyl-D-aspartate receptor antagonists, Psychopharmacology (Berl) 114:573-82.
Gordon RK, Nigam SV, Weitz JA, Dave JR, Doctor BP, Ved HS (2001) The NMDA receptor ion channel: a site for binding of Huperzine A, J Appl Toxicol. 21 Suppl 1:S47-51.
Guitton MJ, Wang J, Puel JL (2004) New pharmacological strategies to restore hearing and treat tinnitus, Acta Otolaryngol 124:411-51.
Hansen RE, Tonsager MW (1988) Determination of the regime of rapid reacting systems in stopped- and steady-flow investigations by the velocity probe method, J Phys Chem 92:2189-2196.
Herin GA, Du S, Aizenman E (2001) The neuroprotective agent ebselen modifies NMDA receptor function via the redox modulatory site, J. Neurochem 78:1307-14.
Hermann A, Varga V, Janaky R, Dohovics R, Saransaari P, Oja SS (2000) Interference of ligands to ionotropic glutamate receptors in pig cerebral cortical synaptic membranes, Neurochem Res 25:1119-24.
Hesselink MB, Smolders H, De Boer AG, Breimer DD, Danysz W (1999) Modifications of the behavioral profile of non-competitive NMDA receptor antagonists, memantine, amantadine and (+) MK-801 after chronic administration, Behav Pharmacol 10:85-98.
Hewitt DJ (2000) The use of NMDA-receptor antagonists in the treatment of pain, Clin J Pain 16:S73-9.
Ikonomidou C, Turski L (2002) Why did NMDA receptor antagonists fail clinical trials for stroke and traumatic brain injury, Lancet Neurol 1:383-6.
Kohl BK, Dannhardt G (2001) The NMDA receptor complex: a promising target for novel anti epileptic strategies, Curr Med Chem 8:1275-89.
Konishi T, Kelsey E (1973) Effect of potassium deficiency on cochlear potentials and cation contents of the endolymph, Acta Otolaryngol 76:410-8.
Kopke RD, Coleman JK, Liu J, Campbell KC, Riffenburgh RH (2002) Candidate's thesis: enhancing intrinsic cochlear stress defenses to reduce noise-induced hearing loss, Laryngoscope 112:1515-32.
Lu Y, Chen SC (2004) Micro and nano-fabrication of biodegradable polymers for drug delivery, Adv Drug Deliv Rev 56:1621-1633.
Morris AW, Morrison GA (1989) Cochlear dialysis for Meniere's disease, An update. Am J. Otol. 10:148-9.
Lynch DR, Gallagher MJ (1996) Inhibition of N-methyl-D-aspartate receptors by haloperidol: developmental and pharmacological characterization in native and recombinant receptors, J Pharmacol Exp Ther 279:154-61.
Lynch DR, Shim SS, Seifert KM, Kurapathi S, Mutel V, Gallagher MJ, Guttmann RP (2001) Pharmacological characterization of interaction of Ro 25-6981 with the NR2B (epsilon2) subunit, Eur J Pharmacol 416:185-95.
Mayer S, Harris BR, Gibson DA, Blanchard JA, Prendergast MA, Holley RC, Littleton J (2002) Acamprosate, MK-801, and ifenprodil inhibit neurotoxicity and calcium entry induced by ethanol withdrawal in organotypic slice cultures from neonatal rat hippocampus, Alcohol Clin Exp Res. 26:1468-78.
Mueller AL, Artman LD, Balandrin MF, Brady E, Chien Y, Delmar EG, George K, Kierstead A, Marriott TB, Moe ST, Newman MK, Raszkiewicz JL, Sanguinetti EL, van Wagenen BC, Wells D (1999) NPS 1506, a novel NMDA receptor antagonist and neuroprotectant. Review of preclinical and clinical studies, Ann N Y Acad Sci. 890:450-7.
Muly SM, Gross JS, Potashner SJ (2004) Noise trauma alters D-[3H]aspartate release and AMPA binding in chinchilla cochlear nucleus, J Neurosci Res 75:585-596.
Nankai M, Fage D, Carter C (1995) NMDA receptor subtype selectivity: eliprodil, polyamine spidertoxins, dextromethorphan, and desipramine selectively block NMDA-evoked striatal acetylcholine but not spermidine release, J Neurochem 64:2043-8.
Ningaraj NS, Chen W, Schloss JV, Faiman MD, Wu JY (2001) S-methyl-N,N-diethylthiocarbamate sulfoxide elicits neuroprotective effect against N-methyl-D-aspartate receptor-mediated neurotoxicity, J Biomed Sci. 2001 8:104-13.
Nordang L, Oestreicher E, Arnold W, Anniko M (2000) Glutamate is the afferent neurotransmitter in the human cochlea, Acta Oto-Laryngologica 120:359-362.
Oestreicher E, Arnold W, Ehrenberger K, Felix D (1998) Memantine suppresses the glutamatergic neurotransmission of mammalian inner hair cells, ORL J Otorhinolaryngol Relat Spec 60:18-21.
Oestreicher E, Arnold W, Ehrenberger K, Felix D (1999) New approaches for inner-ear therapy with glutamate antagonists, Acta Oto-Laryngologica 119:174-178.
Oestreicher E, Ehrenberger K, Felix D (2002) Different action of memantine and caroverine on glutamatergic transmission in the mammalian cochlea, Adv Otorhinolaryngol 59:18-25.
Oliver D, Ludwig J, Reisinger E, Zoellner W, Ruppersberg JP, Fakler B (2001) Memantine inhibits efferent cholinergic transmission in the cochlea by blocking nicotinic acetylcholine receptors of the outer hair cells, Mol Pharmacol 60:183-189.
Orive G, Hernández RM, Gascón AR, Pedraz JL (2005) Micro and nano drug delivery systems in cancer therapy, Cancer Therapy 3:131-138.
Palmer GC (2001) Neuroprotection by NMDA receptor antagonists in a variety of neuropathies, Curr Drug Targets 2:241-71
Parsons CG, Quack G, Bresink I, Baran L, Przegalinski E, Kostowski W, Krzascik P, Hartmann S, Danysz W (1995) Comparison of the potency, kinetics and voltage-dependency of a series of uncompetitive NMDA receptor antagonists in vitro with anticonvulsive and motor impairment activity in vivo, Neuropharmacology 34:1239-58.
Peterson SL, Purvis RS, Griffith JW (2004) Differential neuroprotective effects of the NMDA receptor-associated glycine site partial agonists 1-aminocyclopropanecarboxylic acid (ACPC) and D-cycloserine in lithium-pilocarpine status epilepticus, Neurotoxicology. 25:835-47.
Petty MA, Weintraub PM, Maynard KI (2004) ACEA 1021: flip or flop, CNS Drug Rev 10:337-48.
Popik P, Layer RT, Skolnick P (1994) The putative anti-addictive drug ibogaine is a competitive inhibitor of [3H]MK-801 binding to the NMDA receptor complex, Psychopharmacology (Berl) 114:672-4.
Prakobvaitayakit M, Nimmannit U (2003) Optimization of polylactic-co-glycolic acid nanoparticles containing itraconizole using 23 factorial design, AAPS PharmaSciTech 4(4):E71.
Pujol R, Puel JL (1999) Excitotoxicity, synaptic repair, and functional recovery in the mammalian cochlea: a review of findings, Ann N Y Acad Sci 884:249-254.
Rodrigues CM, Sola S, Nan Z, Castro RE, Ribeiro PS, Low WC, Steer CJ (2003) Tauroursodeoxycholic acid reduces apoptosis and protects against neurological injury after acute hemorrhagic stroke in rats, Proc Natl Acad Sci USA 100:6087-6092.
Rowe R, Sheskey P, Owen S, Eds. (2006) in Handbook of Pharmaceutical Excipients,, APhA Publications Fifth Edition, Washington, DC
Schwarz JB, Gibbons SE, Graham SR, Colbry NL, Guzzo PR, Le VD, Vartanian MG, Kinsora JJ, Letarski SM, Li Z, Dikerson MR, Su TZ, Weber ML, El-Kattan A, Thorpe AJ, Donevan SD, Taylor CP, Wustrow DJ (2005) Novel cyclopropyl beta-amino acid analogues of pregabalin and gabapentin that target the alpha2-delta protein, J Med Chem 48:3026-3035.
Seidman MD, Van De Water TR (2003) Pharmacologic manipulation of the labyrinth with novel and traditional agents delivered to the inner ear, Ear Nose Throat J 82:276-280, 282-3, 287-8.
Stahl SM (2004) Anticonvulsants and relief of chronic pain: pregabalin and gabapentin as alpha(2)delta ligands at voltage-gated calcium channels, J Clin Psychiatry 65:596-597.
Sugimoto M, Uchida I, Mashimo T (2003) Local anaesthetics have different mechanisms and sites of action at the recombinant N-methyl-D-aspartate (NMDA) receptors, Br J Pharmacol 38:876-82.
Takizawa S, Matsushima K, Fujita H, Nanri K, Ogawa S, Shinohara Y (1995) A selective N-type calcium channel antagonist reduces extracellular glutamate release and infarct volume in focal cerebral ischemia, J Cerebral Blood Flow Metab 15:611-8.
Taylor CP (1997) Mechanisms of action of gabapentin, Rev Neurol (Paris) 153 Suppl 1:S39-S45.
Wangemann P, Schacht J (1996) Homeostatic mechanisms in the cochlea, in The Cochlea, Dallos P, Popper AN, Fay RR, Eds., Springer, New York, pp 130-185
Zhou ZL, Cai SX, Whittemore ER, Konkoy CS, Espitia SA, Tran M, Rock DM, Coughenour LL, Hawkinson JE, Boxer PA, Bigge CF, Wise LD, Weber E, Woodward RM, Keana JF (1999) 4-Hydroxy-1-[2-(4-hydroxyphenoxy)ethyl]-4-(4-methylbenzyl)piperidine: a novel, potent, and selective NR1/2B NMDA receptor antagonist, J Med Chem 42:2993-3000.
Zhou LM, Szendirei GI, Fossom LH, Maccecchini ML, Skolnick P, Otvos L Jr (1996) Synthetic analogs of conantokin-G: NMDA antagonists acting through a novel polyamine-coupled site, J Neurochem 66:620-8.

### Paragraphs of the Invention

1. A formulation comprising:
   gacyclidine;
   a vehicle; and
   one or more components selected from the group consisting of a solubility enhancing reagent and an excipient.
2. The formulation of paragraph 1 which comprises a solubility enhancing reagent.
3. The formulation of paragraph 2 wherein the solubility enhancing reagent is a carrier molecule.
4. The formulation of paragraph 3 wherein the carrier molecule is a surfactant.
5. The formulation of paragraph 4 wherein the surfactant is selected from the group consisting of a polysorbate and a poly-oxyethylene ester of 12-hydroxystearic acid.
6. The formulation of paragraph 5 wherein the polysorbate is polysorbate 80.
7. The formulation of paragraph 3 wherein the carrier molecule is a beta-cyclodextrin.
8. The formulation of paragraph 1 which comprises an excipient.
9. The formulation of paragraph 8 wherein the excipient is selected from the group consisting of dimethylsulfoxide (DMSO), dimethyl acetamide, a physiologically acceptable polyol, and an alcohol.
10. The formulation of paragraph 1 wherein the vehicle is physiologically acceptable.
11. The formulation of paragraph 1 wherein gacyclidine is present at a concentration of 0.01 to 5 mM.
12. The formulation of paragraph 1 with an osmolarity of 280-310 mOsm.
13. The formulation of paragraph 1 which has a pH of 5-10.
14. The formulation of paragraph 1 which has a pH of 5.0-6.0.
15. The formulation of paragraph 1 which has a pH of 6.0-7.9.
16. The formulation of paragraph 1 wherein the gacyclidine is a gacyclidine base.
17. The formulation of paragraph 1 wherein the gacyclidine is an acid salt form.
18. The formulation of paragraph 1 wherein the gacyclidine is a mixture of gacyclidine base and a gacyclidine acid salt form.
19. The formulation of paragraph 16, 17, or 18 which comprises a carrier molecule.
20. The formulation of paragraph 19 which comprises an excipient.
21. The formulation of paragraph 1 comprising:
   gacyclidine at a concentration from 1 nM to 5 mM; and
   polysorbate 80 at a concentration from 0.001% to 30% (weight/weight).
22. The formulation of paragraph 1 further comprising a second therapeutic agent.
23. The formulation of paragraph 1 wherein less than 10% of the gacyclidine decomposes at 37°C over 4 days as measured by formation of piperidine.
24. A solid carrier comprising solid gacyclidine free base.
25. The solid carrier of paragraph 24 which is a nanoparticle.
26. The solid carrier of paragraph 25 which is suspended in a physiologically acceptable vehicle.
27. The solid carrier of paragraph 24 which is selected from the group consisting of a collagen sponge, a gel-forming matrix, a gel, a polymeric matrix, and a thin film.
28. The solid carrier of paragraph 24 which is coated onto a device.
29. The solid carrier of paragraph 28 wherein the device is an implantable electrode.
30. A method of making a gacyclidine formulation comprising reconstituting a lyophilized preparation comprising gacyclidine with a vehicle comprising a solubility enhancing reagent.
31. The method of paragraph 30 wherein the solubility enhancing reagent enhances chemical stability of the gacyclidine.
32. A lyophilized preparation of gacyclidine made by lyophilizing a formulation comprising:
   gacyclidine;
   a vehicle; and
   one or more components selected from the group consisting of a solid carrier and an excipient.
33. A method of administering gacyclidine to the middle or inner ear of a mammal, comprising administering to a mammal in need thereof:
   (1) a liquid formulation comprising gacyclidine, a vehicle, and one or more components selected from the group consisting of a solubility enhancing reagent and an excipient; or
   (2) a solid carrier comprising solid gacyclidine free base.
34. The method of paragraph 33 wherein the solid carrier is a nanoparticle suspended in a physiologically acceptable vehicle.
35. The method of paragraph 33 wherein the formulation is administered by direct injection into the middle or inner ear through the round window membrane.
36. The method of paragraph 33 wherein the formulation is administered by direct injection into the inner ear through a cochlear implant electrode.
37. The method of paragraph 33 wherein the formulation is administered using a device selected from the group consisting of a round window catheter, a sponge, a gel, and a wick.
38. The method of paragraph 33 wherein the formulation is administered through direct injection into the middle ear using a needle which penetrates the ear drum.
39. The method of paragraph 33 wherein the formulation is administered through surgical implantation into the middle ear.
40. The method of paragraph 33 wherein the mammal is a human.
41. The method of paragraph 33 wherein the human has a disorder selected from the group consisting of tinnitus, Meniere's disease, vertigo, middle ear inflammation, inner ear inflammation, infection, hearing loss, and neurological damage.
42. The formulation of paragraph 1 comprising:
   gacyclidine at a concentration from 50 µM to 5 mM; and
   polysorbate 80 at a concentration from 0.001% to 30% (weight/weight).

## Claims

1. A pharmaceutical composition for use in treating disorders of the middle and inner ear which comprises ketamine in a gel.

2. A composition for use as claimed in claim 1 in a gel which further comprises a solubility enhancing agent.

3. A composition for use as claimed in claim 1 wherein the gel further comprises hyaluronic acid.

4. A composition for use as claimed in claim 1 wherein the gel further comprises carboxymethylcellulose.

5. A composition for use as claimed in claim 1 wherein the gel further comprises pleuronic acid.

6. A composition for use as claimed in claim 1 which further comprises d-alpha tocopheryl polyethylene glycol 1000 succinate (TPGS), cyclodextrins, ethylene glycol monostearate, glycerol stearate, glycerol mono- / di-caprylate / caprate, glyceryl behenate, glyceryl monooleate, glyceryl monostearate, glyceryl palmitostearate, lecithin, poloxamer 188, polyethylene glycols, polyglyceryl oleate, polyoxyl 40 stearate, polysorbate 20, polyoxyethylene sorbitan fatty acid esters, polyoxyethylene stearates, propylene glycol laurate, sodium lauryl sulfate, sodium stearyl fumarate, sorbitan esters (sorbitan fatty acid esters), sucrose octaacetate, stearic acid.

7. A composition for use as claimed in claim 1 which has a pH of between 6.5 and 8.5.

8. A composition for use a claimed in claim 1 which has as osmolality of 280 - 310 mOsm.
